# EUROPÄISCHE PATENTSCHRIFT

(11) **EP 3 154 931 B1**
(45) Veröffentlichungstag und Bekanntmachung des Hinweises auf die Patenterteilung: **15.08.2018**
(21) Anmeldenummer: 15720079.1
(22) Anmeldetag: 30.04.2015
(51) Int. Cl.: C07C 201/16, C07C 205/06, B01D 11/04, B01D 19/00, B01J 19/24

(54) **VERFAHREN ZUR ENTFERNUNG VON VERUNREINIGUNGEN AUS DINITROTOLUOLEN**
METHOD FOR REMOVING IMPURITIES FROM DINITROTOLUENES
PROCÉDÉ D'ÉLIMINATION DE CONTAMINANTS À PARTIR DE DINITROTOLUÈNES

(30) Priorität: 07.07.2014 DE 102014009948; 25.03.2015 DE 102015003743; 07.04.2015 DE 102015004242
(43) Veröffentlichungstag der Anmeldung: 19.04.2017
(73) Patentinhaber: Josef Meissner GmbH & Co. KG, 50968 Köln (DE)
(72) Erfinder: HERMANN, Heinrich, 50858 Köln (DE); PÖHLMANN, Jürgen, 50969 Köln (DE); HÄNDEL, Mirko, 53819 Neunkirchen-Seelscheid (DE); GEBAUER, Jürgen, 47608 Geldern (DE); BERGMANN, Tim, 51103 Köln (DE)
(74) Vertreter: Von Rohr Patentanwälte Partnerschaft mbB
(86) Internationale Anmeldenummer: PCT/EP2015/059493
(87) Internationale Veröffentlichungsnummer: WO 2016/005070

(56) Entgegenhaltungen:
- EP-A1- 0 897 907
- EP-A2- 1 132 347
- WO-A1-2011/021057

## Beschreibung

Die vorliegende Erfindung betrifft das technische Gebiet der Nitrierung von Toluol zur Herstellung von Dinitrotoluolen mit anschließender Entfernung der Verunreinigungen aus den Rohdinitrotoluolen (Roh-DNTs) zu Zwecken der Bereitstellung von reinen Dinitrotoluolen.

Insbesondere betrifft die vorliegende Erfindung ein Verfahren zur Aufreinigung von aus der Nitrierung (Dinitrierung) (z. B. adiabatische oder isotherme Nitrierung) von Toluol in Gegenwart eines Salpetersäure/Schwefelsäure-Nitiersäuregemisches resultierenden Rohdinitrotoluolen (d. h. also mit anderen Worten ein Verfahren zur Herstellung bzw. Bereitstellung von reinen bzw. aufgereinigten Dinitrotoluolen) sowie eine entsprechende Vorrichtung bzw. Anlage zur Durchführung dieses Verfahrens.

Des Weiteren betrifft die vorliegende Erfindung eine Produktionsanlage zur Herstellung von Dinitrotoluolen (d. h. eine Produktionsanlage zur Nitrierung von Toluol zu Dinitrotoluolen) mit nachfolgender Aufreinigung der bei der Nitrierung entstehenden nitrierten Rohdinitrotoluole (Roh-DNTs).

Dinitrotoluole (DNTs) sind ein wichtiges Zwischenprodukt in der chemischen Industrie, welche nach Reduktion zu den entsprechenden Aminen (z. B. durch Umsetzung mit Wasserstoff in Gegenwart eines Katalysators) vor allem als Ausgangsstoffe bzw. Edukte für Polyurethane dienen.

Im Allgemeinen wird DNT als Isomerengemisch durch Umsetzung von Toluol mit Salpetersäure direkt oder in Gegenwart von Schwefelsäure als Katalysator und Wasser bindendes Mittel einstufig oder in zwei Stufen im Gegenstrom isotherm oder auch adiabatisch hergestellt. Nach Abtrennung der Nitrierendsäure (d. h. im Allgemeinen einem Salpetersäure/Schwefelsäure-Nitrierendsäuregemisch) wird ein noch vielfältig verunreinigtes rohes Dinitrotoluol erhalten, welches erst nach Durchlauf weiterer, meist komplexer Behandlungsschritte in einer Reinheit vorliegt, welche eine Weiterverwendung oder Weiterverarbeitung erlaubt.

Nachfolgend wird im Rahmen der vorliegenden Anmeldung in Bezug auf das bzw. die Dinitrotoluol(e) (DNT bzw. DNTs) synonym die Singular- oder Pluralform verwendet.

Bei der Umsetzung von Toluol zu Dinitrotoluol (DNT) mit Salpetersäure liegen neben den gewünschten Nitrokörpern eine Vielzahl von Verunreinigungen, insbesondere ein Vielzahl von unterschiedlichsten Nebenprodukten und anderweitigen Verunreinigungen, im Roh-DNT vor, hierunter insbesondere noch Spuren an MNT (Mononitrotoluol), TNT (Trinitrotoluol) und Reste von im Ausgangstoluol stets vorhandenen aliphatischen und cycloaliphatischen Kohlenwasserstoffen und daneben eine Reihe von weiteren Nebenprodukten, wie z. B. Mononitrokresole (MNK), Dinitrokresole (DNK) und Trinitokresole (TNK), Trinitrophenol (Pikrinsäure bzw. PS), Nitrobenzoesäuren (NBS), wie Mononitrobenzoesäure (MNBS) und Dinitrobenzoesäure (DNBS), und Abbauprodukte aus der Oxidation der aliphatischen und cycloaliphatischen Kohlenwasserstoffe, der Nitrokresole und Nitroaromaten mit der Salpetersäure aus der Mischsäure, wie z. B. Kohlendioxid (CO₂), Kohlenmonoxid (CO), Blausäure (HCN), Tetranitromethan (TNM), Ameisensäure, Essigsäure, Oxalsäure etc., und Reaktions- bzw. Reduktionsprodukte der Salpetersäure, wie Stickoxide (z. B. Stickstoffoxid NO, Stickstoffdioxid NO₂, Distickstoffoxid N₂O etc.), Salpetrige Säure usw. Zusätzlich befinden sich im rohen DNT noch Salpetersäure und Schwefelsäure, insbesondere in gelöster Form und/oder als Mikroemulsion der Nitrierendsäure in feinverteilter Form.

Im Allgemeinen müssen alle diese Verunreinigungen oder zumindest ein wesentlicher Teil hiervon aus dem rohen DNT vor dessen Weiterverarbeitung oder Weiterverwendung entfernt werden. Das Ausmaß der Reinigung, d. h. der Restgehalt der Verunreinigungen im gereinigten Nitroaromaten, wird insbesondere durch die anwendungsbezogen geforderten Reinheitsgrade bestimmt, welche unter anderem auch von dem für die weitere Verwendung vorgesehenen Verfahren oder dergleichen abhängen. Vor allem müssen alle die Weiterverarbeitung störenden Stoffe, auch in Spuren, auf ein Minimum reduziert werden, wie z. B. die hydrierungsbehindernden Stoffe, wie z. B. schwefelhaltige Verbindungen, Stickoxide, Salpetersäure etc. (vgl. z. B. US 4 224 249 A), Nitrokresole (vgl. z. B. US 2 976 320 A), Kohlendioxid (vgl. EP 1 935 870 A1), Natriumionen oder die bekannten Komplexbildner und Katalysatorgifte, wie z. B. CO, N₂O, Blausäure etc.

Gemäß dem Stand der Technik werden diese Verunreinigungen üblicherweise aus den rohen Nitroaromaten (Roh-DNTs) nach Abtrennung der Nitrierendsäure durch eine Wäsche in mehreren Waschschritten entfernt, bevor der Nitroaromat einer direkten Verwendung, einer Isomerentrennung oder einer Hydrierung zu den entsprechenden Aminen zugeführt wird.

Üblicherweise besteht die Wäsche der rohen Nitroaromaten zur Entfernung der darin gelösten und suspendierten Säuren des Nitriergemisches, der Nitrophenole und anderer saurer und sonst noch mit dem Waschmittel extrahierbaren Verunreinigungen aus drei Schritten (siehe z. B. F. Meissner et al., Industrial and Engineering Chemistry Vol. 46, 721 (1954); Ullmanns Enzyklopädie der technischen Chemie, 4. Auflage. Bd. 17, Seiten 385/386; H. Hermann et al., ACS-Symposium Series 632, 238 (1996), Seite 241 [Herausgeber: L. F. Albright, R. V. C Carr, R. J. Schmitt]; A. B. Quakenbush et al., The Olin Dinitrotoluene (DNT) Process, Polyurethanes World Congress 1993, Publish: Technomic Lancaster, Seiten 484-488; US 6 288 289 B1; EP 1 816 117 A1).

Diese dreistufige Wäsche der rohen Nitroaromaten umfasst im Allgemeinen die folgenden Waschschritte:
1. Waschschritt I oder saure Wäsche:
   Wäsche mit Wasser (sauer eingestellt infolge des Nitrierendsäuregemisches), insbesondere zur Entfernung der gelösten und suspendierten starken Mineralsäuren, wie Schwefelsäure und Salpetersäure, und der Nitrose.
2. Waschschritt II oder Alkaliwäsche (basische Wäsche):
   Wäsche in Gegenwart einer Base, wie z.B. Natriumcarbonat (Soda), Natriumbicarbonat, Natriumsulfit bzw. Natriumhydrogensulfit, Ammoniak, Natronlauge, Kalilauge etc. (vgl. z. B. US 4 482 769 A, US 4 597 875 A oder US 6 288 289 B1), insbesondere zur Entfernung von in den Nitroaromaten nach Waschschritt I noch gelösten und suspendierten Spuren an Salpetersäure, Stickoxiden und Schwefelsäure und von noch in den Nitroaromaten gelösten schwach aciden Verunreinigungen, wie Nitrophenolen, Nitrokresolen, Nitrobenzoesäuren, Abbauprodukten aus der oxidativen Zersetzung von Phenolen oder aliphatischen und/oder cyclischen Kohlenwasserstoffen etc., wie z.B. Blausäure, Oxalsäure, Ameisensäure, Essigsäure etc.
3. Waschschritt III oder Neutralwäsche:
   Neutralwäsche, insbesondere zur Entfernung der Restspuren von Alkalien bzw. basischen Verbindungen aus Mikroemulsion oder sonstigem Mitriss aus Waschschritt II und zur weiteren Reduzierung der noch in Spuren im Produkt verbliebenen Verunreinigungen.

Dabei kann grundsätzlich jeder Waschschritt bis zu 10 Extraktionsstufen im Quer- oder Gegenstrom oder einer Kombination daraus umfassen, wie beispielsweise in der CA 1 034 603, in der EP 1 780 195 A1, in der US 6 288 289 B1 oder in der WO 2013/160367 A1 beschrieben.

Als Waschmedium wird für die Wäsche üblicherweise Wasser eingesetzt. Im Allgemeinen wird dabei die Wäsche als flüssig/flüssig-Extraktion durchgeführt, d. h. also bei Temperaturen, bei welchen der zu waschende Nitroaromat als Flüssigkeit vorliegt.

Ziel dieser im Allgemeinen mehrstufigen Wäsche ist es, neben einem reinen Produkt, welches ohne negative Beeinflussung durch noch vorhandene Verunreinigungen weiterverarbeitet werden kann, möglichst wenig Abwasser pro Tonne Produkt zu erhalten, indem die ausgewaschenen Verunreinigungen in einer solchen Weise vorliegen, dass ihre Entsorgung kostengünstig durchgeführt werden kann, wobei nichtumgesetzte Edukte vorteilhafterweise zurückgewonnen bzw. rezykliert werden.

Bei der Wäsche der Roh-DNTs mit Wasser im Rahmen einer flüssig/flüssig-Extraktion werden aber vorwiegend nur diejenigen Verunreinigungen ausgewaschen, welche eine hohe Löslichkeit im Waschmedium besitzen (wie z. B. Schwefelsäure und Salpetersäure) oder welche im wässrigen Medium mit dem Wasser zu Salpetersäure disproportionieren oder welche mit den im Rahmen der alkalischen bzw. basischen Wäsche (siehe obiger Waschschritt II) zugesetzten Basen Salze bilden und damit irreversibel aus ihrem Verteilungsgleichgewicht mit der DNT-Organphase entfernt werden (wie z. B. die Restspuren der starken Säuren [Schwefelsäure und Salpetersäure] und der mittelstarken und schwachen Säuren [wie z. B. Nitrophenole, Nitrokresole, Nitrobenzoesäuren, Essigsäure, Oxalsäure, Ameisensäure, Kohlensäure, Blausäure etc.]). Selbst aber in Bezug auf diese Verunreinigungen ist deren Entfernung im Rahmen der Wäsche nicht immer vollständig bzw. quantitativ, insbesondere dann nicht, wenn die Roh-DNTs hohe Beladungen an Verunreinigungen aufweisen.

Auch hat es sich ferner gezeigt, dass sich anderweitige Verunreinigungen, insbesondere Verunreinigungen mit ungünstigen Verteilungskoeffizienten in Bezug auf das Waschmedium, Verunreinigungen mit schwacher oder fehlender Acidität und Verunreinigungen mit langsamer Umwandlungsgeschwindigkeit in solche Stoffe, welche sich in der Organphase nicht lösen, mit einer Wäsche mit einem oder mehreren Schritten im Rahmen einer flüssig/flüssig-Extraktion oftmals nicht vollständig und/oder nur mit großem technischen Aufwand entfernt werden können. Dies gilt vor allem dann, wenn aus verfahrensökonomischen Gründen (insbesondere zur Reduzierung der Abwasserfracht) die Wäsche in den einzelnen Waschschritten mit einem Verhältnis von eingesetztem Waschmedium zu waschendem Nitroaromat von kleiner 1 (z. B. bei einem Volumenverhältnis im Bereich von 1 : 5 bis 1 : 20) erfolgt, und zwar selbst dann, wenn das Verhältnis Waschmedium zu Nitroaromat im Waschapparat durch Kreisführung des Waschmediums 1 beträgt oder sogar größer als 1 ist. Zu solchen im Rahmen einer Wäsche nur schwer oder nur unvollständig entfernbaren Verunreinigungen gehören typischerweise, aber nicht beschränkend Stickoxide NOx (z. B. Stickstoffoxid NO, Stickstoffdioxid NO₂, Distickstoffoxid N₂O etc.), Blausäure, aliphatische und aromatische Kohlenwasserstoffe, Kohlenstoffoxide (CO, CO₂ etc.) etc.

Insbesondere erweist sich die Entfernung der Verunreinigungen aus den Roh-DNTs dann als schwierig, wenn nur ein Waschschritt zum Einsatz kommt, z. B. nur eine saure Wäsche. Wie in der CA 1 034 603, der US 4 224 249 A, der EP 0 297 312 A1, der EP 0 736 514 A1 oder der EP 1 780 195 A1 beschrieben, enthält das gewaschene DNT neben Spuren der starken Säuren Schwefelsäure, Salpetersäure und Stickoxide NOx noch alle mittelstarken und schwachen organischen und anorganischen Säuren mit pKₛ-Werten, welche gleich oder größer als der pH-Wert der Waschsäure sind, wie z. B. Nitrokresole, Nitrobenzoesäuren, Blausäure, CO₂ etc., und darüber hinaus auch alle neutralen bzw. nichtaciden Verunreinigungen, wie z. B. CO, N₂O, Kohlenwasserstoffe etc., welche entsprechend ihrer Löslichkeit in der Waschsäure und in Abhängigkeit von ihrem individuellen Verteilungsgleichgewicht nur teilweise ausgewaschen werden und damit weiterhin im DNT verbleiben. Besonders schwierig ist dabei die Abtrennung der Salpetersäure, da aus dem stets noch im DNT in geringer Mengen gelösten NOx in Gegenwart von Spuren dispergiertem Wasser stets Salpetersäure neu gebildet wird.

Nach der CA 1 034 603 werden sieben Wäschen mit Wasser im Querstrom (Beispiel 2) benötigt, um einen Restgehalt an Mineralsäuren im DNT von ca. 15 ppm zu erreichen. Die mittelstarken und schwachen anorganischen und organischen Säuren verbleiben bei pH-Werten kleiner 4 (nach 7 Wasserwäschen) in der Organphase und werden nur entsprechend ihren Verteilungsgleichgewichten in das Waschmedium extrahiert.

Gemäß der US 4 224 249 A enthält ein nur mit Wasser gewaschenes DNT weniger als 6.000 ppm Gesamtacidität und bevorzugt weniger als 3.000 ppm (berechnet als Schwefelsäure) an Säuren. Davon sind 1.100 ppm Nitrat, 160 ppm Nitrit und 230 ppm Sulfat. Alle schwachen Säuren, wie Nitrokresole, und alle Neutralverunreinigungen verbleiben dagegen im DNT.

In der EP 0 297 312 A1, der EP 0 736 514 A1 und der EP 1 780 195 A1 werden Verfahren offenbart, welche es gestatten, speziell den Waschschritt der sauren Wäsche derart durchzuführen, dass eine Waschsäure mit möglichst hohen Konzentrationen an Säure (einem Gemisch aus Schwefelsäure, Salpetersäure und Salpetriger Säure bzw. NOx) erhalten wird. Diese Waschsäure wird direkt oder vorzugsweise nach Aufkonzentrierung auf eine Gesamtsäure (berechnet als Salpetersäure) von 50 % bis 60 % in die Nitrierung zurückgeführt. Um diese Anreicherung der Schwefel- und Salpetersäure in der Waschsäure zu erreichen, muss das Verhältnis von DNT zu Frischwasser sehr groß sein. So wird in dem Ausführungsbeispiel der EP 0 736 514 A1 eine Tonne Roh-DNT mehrstufig im Gegenstrom mit ca. 110 Litern Frischwasser (entsprechend einem Verhältnis von DNT zu Frischwasser von 9,1 : 1) gewaschen, um eine Waschsäure mit ca. 23,3 % an Gesamtsäure zu erhalten (entsprechend einer Menge an Waschsäure von ca. 142 kg pro Tonne DNT). Mit einem Verhältnis von DNT zu Frischwasser für die saure Wäsche von ca. 13 : 1 würden nur ca. 77 Liter Frischwasser pro Tonne zu waschendem Roh-DNT benötigt, und man würde eine Waschsäure mit mehr als 30 % Gesamtsäure erhalten.

Bei einem solchen Phasenverhältnis von DNT zu Wasser von ca. 9 : 1 bzw. von 13 : 1 wird mit Verunreinigungen mit Extraktionsfaktoren ε von 1 und kleiner und Verteilungskoeffizienten zugunsten des Extraktionsmittels von 10 und kleiner eine erschöpfende Extraktion mit Extraktionsausbeuten von 90 % und mehr nur infolge einer sehr hohen bis theoretisch unendlichen Anzahl von Extraktionsstufen ermöglicht (vgl. hierzu z. B. K. Sattler, Thermische Trennverfahren, Verlag Wiley-VCH, 3. Auflage 2001, Seiten 545 ff.); dies ist technisch extrem aufwendig und sehr kostenintensiv und zudem bei Verunreinigungen mit Verteilungskoeffizienten kleiner 5 technisch nicht mehr möglich.

Dagegen können mit einer solchen herkömmlichen ein- oder mehrstufigen Wäsche in Form einer flüssig/flüssig-Extraktion, wie sie beispielsweise im Stand der Technik gemäß der EP 0 297 312 A1, der EP 0 736 514 A1 oder der EP 1 780 195 A1 beschrieben ist, Verunreinigungen mit ungünstigen Verteilungskoeffizienten in Bezug auf das Waschmedium, Verunreinigungen mit geringer Löslichkeit im Waschmedium, Verunreinigungen mit schwacher oder fehlender Acidität und Verunreinigungen mit langsamer Umwandlungsgeschwindigkeit in Stoffe, welche sich in der Organphase nicht lösen, nicht bzw. nicht vollständig und nicht effizient aus dem Roh-DNT entfernt werden. Zu den auf diese Weise nicht entfernbaren Verunreinigungen gehören beispielsweise (aber nicht beschränkend) Stickoxide NOx, Kohlenstoffoxide wie CO und CO₂, Blausäure, Kohlenwasserstoffe etc.

Auch mit einer Wäsche von Roh-DNTs in drei Waschschritten mit jeweils bis zu fünf Extraktionsstufen nach dem Stand der Technik, wobei ein Waschschritt in Gegenwart einer Base, bevorzugt Soda, bei einem pH-Wert im Bereich von 8,5 bis 9,2 durchgeführt wird, werden nur schwache Mineralsäuren, wie Kohlensäure, und Nitrophenole sowie Nitrokresole und organischen Säuren, wie Nitrobenzoesäuren, Essigsäure, Oxalsäure, Ameisensäure etc., mit pKₛ-Werten unterhalb von 7 mehr oder weniger quantitativ ausgewaschen. Aber schon Blausäure mit einem pKₛ-Wert von ca. 9,2 wird bei der üblichen Wäsche mit Soda im pH-Bereich von 8,5 bis 9,2 nur teilweise aus dem Roh-DNT extrahiert; die aromatischen und aliphatischen Kohlenwasserstoffe sowie CO, N₂O etc. können durch Extraktion nur entsprechend ihren Löslichkeiten und Verteilungsgleichgewichten aus dem Roh-DNT entfernt werden. Entsprechendes gilt, wenn als Base Ammoniak im Überschuss eingesetzt wird; Spuren desselben können dann noch zusätzlich im gewaschenen DNT vorliegen.

Eine Wäsche zur Verbesserung des Extraktionsgrades von Verunreinigungen mit ungünstigen Verteilungskoeffizienten und/oder mit geringer Löslichkeit im Waschmedium oder von Verunreinigungen mit schwacher oder fehlender Acidität (wie CO₂, Blausäure, CO, N₂O etc.) oder von flüchtigen bzw. niedrigsiedenden aliphatischen und aromatischen Kohlenwasserstoffen (wie Hexan, Pentan, Cyclohexan, Methylcyclohexan, Dimethylcyclohexan, Toluol etc.) oder von Verunreinigungen mit langsamen Umwandlungsgeschwindigkeiten in Stoffe, welche sich in der Organphase nicht mehr lösen (wie NOx bei niedrigen Konzentrationen etc.), erfordern eine hohe Anzahl von Wasch- bzw. Extraktionsstufen im Gegenstrom oder auch im Querstrom oder einer Kombination daraus, verbunden mit einem unverhältnismäßig hohem Mehrverbrauch an Waschmedium und folglich einem Mehranfall von Abwasser, was wiederum zu nicht akzeptablen Investitions- und Betriebskosten führt.

Weiterhin wird in der EP 0 897 907 B1 ein Verfahren zur Rückgewinnung von Salpetersäure in eine Mischung von aromatischen Verbindungen beschrieben, wobei zwei sich ergänzende Verfahrensschritte zur Reinigung der Rohnitrierprodukte eingesetzt werden sollen: Das Rohnitrierprodukte werden nach der EP 0 897 907 B1 vor der eigentlichen Wäsche einer Destillation oder einem Abstrippen zur Entfernung der Salpetersäure unterworfen, um die in den Rohnitrierprodukten gelöste oder suspendierte Salpetersäure vor der Wäsche teilweise separat abzutrennen und nach entsprechender Behandlung zu rezyklieren. Dieses Verfahren benötigt aber gegenüber dem zuvor geschilderten Stand der Technik zur Reinigung von Rohnitrierprodukten erheblichen zusätzlichen technischen Aufwand, ohne dass eine gegenüber dem zuvor geschilderten Stand der Technik verbesserte Produktreinheit erreicht werden kann. Neben dem Abfallstrom aus der stets noch notwendigen nachfolgenden Wäsche (d. h. im Allgemeinen zwei Abfallströme aus der Wäsche, nämlich Waschsäure aus der sauren Wäsche und Waschlauge aus der basischen Wäsche) wird durch den vorgeschalteten Schritt der Destillation oder Abstrippung ein zusätzlicher, dritter Abfallstrom mit verdünnter Salpetersäure erzeugt, welcher getrennt aufbereitet werden muss. Werden der Abfallstrom aus der Wäsche und aus der vorgelagerten Destillations- bzw. Abstrippungsstufe zu Zwecken der gemeinsamen Weiterverarbeitung vereinigt, ist neben dem erhöhten technischen Aufwand kein Vorteil mehr im Vergleich zu einer Wäsche nach Stand der Technik gegeben. Die vorgeschaltete Destillation bzw. Abstrippung dient zudem ausschließlich der Salpetersäureentfernung bzw. -rückgewinnung, ermöglicht aber keine Verbesserung in Bezug auf die Entfernung der schwer entfernbaren Verunreinigungen aus den Rohnitrierprodukten. Auch wird nicht auf die Besonderheiten, wie sie speziell bei der Aufreinigung von Rohdinitrotoluolen auftreten, fokussiert, sondern nur ganz allgemein auf die Dinitrierung beliebiger aromatischer Verbindungen abgestellt. Weiterhin stellt das in der EP 0 897 907 B1 vorgeschlagene Verfahren ein erhöhtes Sicherheitsrisiko im Vergleich zum Stand der Technik dar und ist daher insbesondere speziell für die Aufreinigung von Roh-DNTs ungeeignet: Bei einem Gehalt von bis zu 10 % an Salpetersäure in den Rohnitrierprodukten und bei Temperaturen bis zu 130 °C bei der vorgeschlagenen Destillation sind oxidative und unkontrollierte Zersetzungen der in den Rohnitrierprodukten vorliegenden Verunreinigungen (wie z. B. Nitrokresolen, Nitrobenzoesäuren etc.) wie auch des DNT selbst nicht sicher auszuschließen. Das dort beschriebene Verfahren ist folglich nicht für die effiziente und sichere Aufreinigung speziell von Rohdinitrotoluolen geeignet. Insbesondere können mit dem dort beschriebenen Verfahren keine Spurenverunreinigungen entfernt werden, um hochreines DNT zu erzeugen.

Die EP 1 132 347 A2 betrifft ein integriertes Verfahren zur Behandlung des Abwassers von alkalischem Waschwasser aus der Nitroaromatenherstellung, wobei das dort beschriebene Verfahren alkalisches Waschwasser zur Rückgewinnung von Chemikalien und Wasser vor der Behandlung des Konzentrats durch eine Thermoabbau-Oxidationsbehandlung konzentriert, um auf diese Weise zu verringertem Chemikalien-Wasserverbrauch zu führen.

Die WO 2011/021057 A1 betrifft ein Waschsystem für nitroaromatische Verbindungen, insbesondere ein Verfahren zur Entfernung von Alkalinität und Salzen aus nitroaromatischen Produkten stromabwärts in Bezug auf die Wäsche zur Entfernung der Mineralsäuren und der alkalischen Wäsche zur Entfernung von Salzen organischer Säuren, wobei der Rohproduktstrom zunächst mit einer sauren wässrigen Waschlösung gewaschen wird, bevor überschüssige organische Reagenzien durch Wasserdampfbehandlung oder Destillation entfernt werden.

Die EP 0 897 907 A1 betrifft ein Verfahren zur Rückführung von Salpetersäure in eine Mischung von aromatischen Dinitroverbindungen, wobei zunächst eine Destillation oder eine Wasserdampf-Strippung des nitrierten Rohprodukts erfolgt, gefolgt von einer Wäsche.

Aus dem Stand der Technik ist also insgesamt kein Verfahren bekannt, mit welchem sich in effizienter, verfahrensökonomischer und technisch einfacher Weise Verunreinigungen aller Art aus Rohdinitrotoluolen (Roh-DNTs), welche aus der Dinitrierung von Toluol in Gegenwart eines Salpetersäure/Schwefelsäure-Nitriersäuregemisches hervorgehen, vollständig entfernt lassen, insbesondere im Rahmen einer herkömmlichen Wäsche nur schwer oder nur unvollständig entfernbare Verunreinigungen (d. h. insbesondere Verunreinigungen mit ungünstigen Verteilungskoeffizienten in Bezug auf das Waschmedium, Verunreinigungen mit schwacher oder fehlender Acidität und Verunreinigungen mit langsamer Umwandlungsgeschwindigkeit in solche Stoffe, welche sich in der Organphase nicht lösen).

Eine Aufgabe der vorliegenden Erfindung ist es daher, ein Verfahren bzw. eine Vorrichtung (Anlage) zur Aufreinigung von aus der Nitrierung von Toluol resultierenden Rohdinitrotoluolen (Roh-DNTs) bereitzustellen, wobei die zuvor geschilderten Nachteile und Unzulänglichkeiten des Standes der Technik zumindest weitgehend vermieden oder aber wenigstens abgeschwächt werden sollen. Durch die vorliegende Erfindung sollen in technisch effizienter wie sicherer und einfacher Art und Weise hochreine bzw. aufgereinigte Dinitrotoluole (DNTs) bereitgestellt werden können. Insbesondere sollen mit der vorliegenden Erfindung auch schwer entfernbare Verunreinigungen, insbesondere flüchtige Verunreinigungen, wirksam aus den Roh-DNTs entfernt werden können.

Insbesondere ist eine Aufgabe der vorliegenden Erfindung darin zu sehen, ein Verfahren und eine zur Durchführung dieses Verfahrens geeignete Vorrichtung bzw. Anlage bereitzustellen, mit welchem bzw. welcher eine effiziente Aufreinigung der nitrierten Rohprodukte, wie sie aus der Nitrierung (Dinitrierung) von Toluol in Gegenwart eines Salpetersäure/Schwefelsäure-Nitriersäuregemisches nach Abtrennung des sogenannten Nitrierendsäuregemisches hervorgehen, ermöglicht werden soll.

Weiterhin ist eine Aufgabe der vorliegenden Erfindung darin zu sehen, ein Verfahren und eine Vorrichtung zur Durchführung dieses Verfahrens bereitzustellen, welches bzw. welche es ermöglichen soll, aus einer adiabatischen oder isothermen Dinitrierung von Toluol hervorgehende Rohdinitrotoluole (Roh-DNTs) auf sichere Weise und mit möglichst geringem technischen Aufwand derart aufzureinigen, dass der Gehalt an Verunreinigungen in den DNTs so reduziert wird, dass die für eine technische DNT-Qualität (sogenanntes *"Technical Grade DNT"*) gegebenen Spezifikationen erreicht bzw. möglichst unterschritten werden. Insbesondere soll durch ein solches Verfahren bzw. eine solche Vorrichtung ermöglicht werden, dass selbst die nach der Wäsche der Roh-DNTs mit einem Waschmedium immer noch vorliegenden beträchtlichen Mengen an zum Teil nur aufwendig abtrennbaren Verunreinigungen (z. B. leichtflüchtige Verunreinigungen, wie Stickoxide, Salpetersäure, Salpetrige Säure, Blausäure etc.) in effizienter Weise entfernt werden können.

Die zuvor geschilderte Aufgabenstellung wird erfindungsgemäß durch ein Verfahren nach Anspruch 1 gelöst; weitere, vorteilhafte Weiterbildungen und Ausgestaltungen des erfindungsgemäßen Verfahrens sind Gegenstand der diesbezüglichen Unteransprüche.

Weiterer Gegenstand der vorliegenden Erfindung ist eine Vorrichtung bzw. Anlage nach Anspruch 7; weitere, vorteilhafte Weiterbildungen und Ausgestaltungen dieses Erfindungsaspekts sind Gegenstand der diesbezüglichen Unteransprüche.

Darüber hinaus ist Gegenstand der vorliegenden Erfindung eine Produktionsanlage gemäß Anspruch 10; weitere, vorteilhafte Ausgestaltungen und Weiterbildungen dieses Aspektes sind Gegenstand des diesbezüglichen Unteranspruchs.

Es versteht sich von selbst, dass Ausgestaltungen, Ausführungsformen, Vorteile oder dergleichen, welche nachfolgend - zu Zwecken der Vermeidung von unnötigen Wiederholungen - nur zu einem Erfindungsaspekt ausgeführt werden, selbstverständlich auch in Bezug auf alle übrigen Erfindungsaspekte entsprechend gelten.

Weiterhin versteht es sich von selbst, dass bei der nachfolgenden Angabe von Werten, Zahlen und Bereichen die diesbezüglichen Werte-, Zahlen- und Bereichsangaben nicht beschränkend zu verstehen sind; es versteht sich für den Fachmann von selbst, dass einzelfallbedingt oder anwendungsbezogen von den angegebenen Bereichen bzw. Angaben abgewichen werden kann, ohne dass der Rahmen der vorliegenden Erfindung verlassen ist.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungs- bzw. Analysemethoden ermittelt bzw. bestimmt werden können.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist somit ein Verfahren zur Aufreinigung von aus der Nitrierung (d. h. Dinitrierung) von Toluol in Gegenwart eines Salpetersäure/Schwefelsäure-Nitriersäuregemisches resultierenden Rohdinitrotoluolen, insbesondere zur Entfernung auch von insbesondere flüchtigen Verunreinigungen aus den Rohdinitrotoluolen, wobei bei dem Verfahren
(a) die Rohdinitrotoluole nach Abtrennung des Nitrierendsäuregemisches zunächst einer Wäsche mit mindestens einem Waschmedium unterzogen werden, gefolgt von einer Abtrennung des Waschmediums (d. h. also des verbrauchten bzw. gebrauchten bzw. mit Verunreinigungen beladenen Waschmediums); und
(b) nachfolgend die aus Verfahrensschritt (a) resultierenden gewaschenen Dinitrotoluole einer Strippung (Strippgasbehandlung) mit mindestens einem Gas (Strippgas) unterzogen werden, wobei als Strippgas ein gegenüber Dinitrotoluolen nichtreaktives und/oder gegenüber Dinitrotoluolen inertes Gas eingesetzt wird, wobei das Strippgas ausgewählt ist aus der Gruppe von Stickstoff, Sauerstoff, Edelgasen, Wasserstoff, Kohlenstoffoxiden und deren Mischungen, und wobei die Strippung in Abwesenheit von Wasserdampf durchgeführt wird.

Das erfindungsgemäße Verfahren eignet sich in hervorragender Weise zur Aufreinigung von aus der Nitrierung (Dinitrierung) von Toluol in Gegenwart eines Salpetersäure/Schwefelsäure-Nitriersäuregemisches resultierenden Rohdinitrotoluolen (Roh-DNTs), insbesondere zur effizienten Entfernung auch von ansonsten nur schwer entfernbaren, insbesondere flüchtigen, schwach aciden oder neutralen Verunreinigungen mit einem im Vergleich zum DNT niedrigen Siedepunkt (wie z. B. Stickoxiden wie Stickstoffmonoxid N₂O unter anderen, Kohlenmonoxid, Kohlendioxid, Blausäure bzw. Cyanwasserstoff, Tetranitromethan, aliphatischen und aromatischen Kohlenwasserstoffen etc.). Aber auch anderweitige Verunreinigungen, welche zum Teil nur im Spurenbereich vorliegen und im Rahmen der Wäsche nicht bzw. nur zum Teil bzw. nur unvollständig entfernt werden können (wie z. B. Salpetersäure, Wasser etc.), werden in effizienter Weise entfernt.

Es versteht sich von selbst, dass im Rahmen des erfindungsgemäßen Gesamtverfahrens zumindest im Wesentlichen sämtliche in den Rohdinitrotoluolen vorliegenden Verunreinigungen entfernt werden können, und zwar unabhängig davon, um welche Verunreinigungen es sich handelt, insbesondere unabhängig von deren Flüchtigkeiten, Aciditäten, Verteilungs- oder Exktraktionskoeffizienten in Bezug auf das Waschmedium oder dergleichen. Zu den typischen, nicht beschränkend aufgezählten Verunreinigungen, welche in den aus der Toluolnitrierung hervorgehenden Rohdinitrotoluolen vorliegen und welche im Rahmen des erfindungsgemäßen Verfahrens in effizienter Weise zumindest im Wesentlichen vollständig entfernt werden können, gehören beispielsweise Spuren der im zu nitrierenden Ausgangstoluol stets vorhandenen aliphatischen und cycloaliphatischen Kohlenwasserstoffe sowie deren oxidativen Abbauprodukte, Nitrokresole (Mono-, Di- und Trinitrokresole), Trinitrophenole bzw. Pikrinsäure, Nitrobenzoesäuren (Mono- und Dinitrobenzoesäure) sowie Abbauprodukte aus der Oxidation der vorgenannten Kohlenwasserstoffe, der Nitrokresole und Nitroaromaten, wie z. B. Kohlenstoffoxide (Kohlenmonoxid und Kohlendioxid), Blausäure, Tetranitromethan, Ameisensäure, Essigsäure, Oxalsäure etc., und darüber hinaus Reaktionsprodukte der Salpetersäure, wie Stickoxide (Stickstoffmonoxid, Distickstoffmonoxid, Stickstoffdioxid etc.) usw.; darüber hinaus befinden sich in den Rohdinitrotoluolen zusätzlich noch Salpetersäure, Salpetrige Säure und Schwefelsäure, insbesondere in gelöster oder emulgierter bzw. feinverteilter Form.

Im Rahmen der vorliegenden Erfindung können in effizienter und verfahrensökonomischer Weise nicht nur diejenigen Verunreinigungen entfernt werden, welche eine hohe Löslichkeit im Waschmedium besitzen oder welche im wässrigen Medium mit dem Wasser zu Salpetersäure disproportionieren oder welche mit den im Rahmen der alkalischen bzw. basischen Wäsche zugesetzten Basen Salze bilden und damit irreversibel aus ihrem Verteilungsgleichgewicht mit der DNT-Organphase entfernt werden, sondern darüber hinaus auch die im Rahmen einer herkömmlichen Wäsche nur schwer oder nur unvollständig entfernbaren Verunreinigungen, nämlich insbesondere Verunreinigungen mit ungünstigen Verteilungskoeffizienten in Bezug auf das Waschmedium, Verunreinigungen mit schwacher oder fehlender Acidität und Verunreinigungen mit langsamer Umwandlungsgeschwindigkeit in solche Stoffe, welche sich in der Organphase nicht lösen.

Alle vorgenannten Verunreinigungen können im Rahmen des erfindungsgemäßen Verfahrens zumindest im Wesentlichen vollständig entfernt werden, wobei die Anmelderin überraschend herausgefunden hat, dass dies nur möglich ist, wenn der erfindungsgemäß vorgesehene Verfahrensschritt (b) der Strippgasbehandlung bzw. Strippung zusätzlich zu einer Wäsche der Rohdinitrotoluole gemäß Verfahrensschritt (a) durchgeführt wird und wenn vor allem die Strippgasbehandlung der Wäsche der rohen Dinitrotoluole nachgeschaltet wird. Wie die Anmelderin gleichermaßen überraschend gefunden hat, führt eine Umkehrung der Durchführung der beiden Verfahrensschritte dagegen nicht zu den gewünschten Ergebnissen (d. h. es findet in diesem Fall keine effiziente Entfernung aller Verunreinigungen statt). Nur mit einer der Wäsche nachgeschalteten Strippgasbehandlung kann überraschenderweise auch eine Entfernung von Spurenverunreinigungen zum Erhalt von Rein-DNTs erreicht werden.

Das Prinzip des erfindungsgemäßen Verfahrens besteht also darin, die aus der Nitrierung stammenden, noch signifikante Mengen an Verunreinigungen enthaltenden rohen Dinitrotoluole - nach Abtrennung der Nitrierendsäure bzw. des Nitrierendsäuregemisches (z. B. in einem Scheider etc.) - zunächst einer Wäsche mit einem Waschmedium mit nachfolgender Abtrennung des Waschmediums zu unterziehen und die auf diese Weise gewaschenen Dinitrotoluole nachfolgend einer Strippgasbehandlung (d. h. einer Strippung mit mindestens einem Gas bzw. Strippgas) zu unterwerfen.

Denn, wie die Anmelderin in vollkommen überraschender Weise herausgefunden hat, führt die Durchführung einer der Wäsche nachgeschalteten Strippung dazu, dass zumindest im Wesentlichen sämtliche nach der Wäsche noch vorhandenen Verunreinigungen, insbesondere ansonsten nur schwer entfernbare Verunreinigungen, wie z. B. die vorgenannten leichtflüchtigen Verunreinigungen, in effizienter, einfacher und sicherer Weise entfernt werden können. Wie die Anmelderin gleichermaßen gefunden hat, liegen selbst nach einer in mehreren Schritten durchgeführten Wäsche der Rohdinitrotoluole immer noch beträchtliche Mengen an zum Teil nur aufwändig abtrennbaren Verunreinigungen in den Dinitrotoluolen vor, welche erfindungsgemäß durch die Strippung wirksam entfernt werden können.

Wie die Anmelderin ebenfalls vollkommen überraschend herausgefunden hat, bewirkt nur die erfindungsgemäße Kombination von vorgeschalteter Wäsche und nachgeschalteter Strippgasbehandlung (Strippung) eine effiziente Aufreinigung von aus der Nitrierung von Toluol resultierenden Rohdinitrotoluolen - und dies auch nur, wenn in der erfindungsgemäßen Reihenfolge der Verfahrensschritte vorgegangen wird (d. h. die Strippung der Waschbehandlung bzw. dem Wäscheschritt nachgeschaltet wird). Überraschend lassen sich auf diese Weise selbst Spurenverunreinigungen von ansonsten nicht oder allenfalls nur sehr aufwendig entfernbaren Verunreinigungen (z. B. Blausäure etc.) zumindest im Wesentlichen vollständig entfernen.

Im Unterschied zum Stand der Technik werden weitergehende, komplexe Verfahrensschritte zur Aufreinigung der rohen Dinitrotoluole in effizienter Weise vermieden, ohne dass Qualitätseinbußen bei der Aufreinigung der Rohdinitrotoluole hinzunehmen wären.

Überraschenderweise gewährleistet die der Wäsche nachgeschaltete Strippgasbehandlung bzw. Strippung eine sichere wie einfache bzw. effiziente Entfernung noch vorhandener Verunreinigungen, und zwar selbst im Spurenbereich. Darüber hinaus bewirkt die Strippgasbehandlung bzw. Strippung gleichermaßen (in Abhängigkeit von der Beladung des Strippgases mit Wasser) eine mehr oder weniger umfangreiche Entfernung des in den zu behandelnden Dinitrotoluolen noch vorhandenen (z. B. gelösten oder suspendierten) Restwassers, d. h. dieses Restwasser kann wirksam entfernt bzw. zumindest signifikant reduziert werden.

Im Rahmen der Erfindung wird also ein einfach zu handhabendes Verfahren zur Aufreinigung von aus der Nitrierung von Toluol resultierenden Rohdinitrotoluolen bereitgestellt. Mit anderen Worten wird im Rahmen der vorliegenden Erfindung ein Verfahren zur Bereitstellung bzw. Herstellung aufgereinigter bzw. reiner (hochreiner) Dinitrotoluole (DNTs) zur Verfügung gestellt.

Gemäß einer typischen Ausführungsform der vorliegenden Erfindung betrifft die vorliegende Erfindung somit insbesondere ein Verfahren zur Gewinnung von reinem Dinitrotoluol (DNT) als Isomerengemisch oder aber auch als reines Isomeres ausgehend von Roh-DNT, welches aus einer isothermen oder adiabatischen Umsetzung von Toluol mit Salpetersäure in Gegenwart von Schwefelsäure erhalten wird. Dabei wird nach Abtrennung der Nitrierendsäure das Roh-DNT durch eine Behandlung mit einem Waschmedium (im Allgemeinen Wasser) in ein oder mehreren Waschschritten in Kontakt gebracht und auf diese Weise gewaschen (wobei typischerweise im Fall mehrerer Waschschritte auch ein Waschschritt in Gegenwart einer Base, insbesondere bei einem pH-Wert im Bereich von 8 bis 12, durchgeführt wird) und nachfolgend - nach Abtrennung des Waschmediums - das mit Wasser gewaschene DNT mit einem Strippgas behandelt wird.

Was Verfahrensschritt (a) (d. h. den Waschschritt) des erfindungsgemäßen Verfahrens anbelangt, so ist diesbezüglich Folgendes auszuführen:
Üblicherweise wird die Wäsche als sogenannte flüssig/flüssig-Extraktion durchgeführt.

Bei der vorliegenden Erfindung kann im Rahmen des erfindungsgemäßen Verfahrens in Verfahrensschritt (a) die Wäsche in ein oder mehreren Waschschritten, insbesondere in ein, zwei oder drei Waschschritten, durchgeführt werden. Dabei kann jeder Waschschritt wiederum ein oder mehrere Wasch- und/oder Extraktionsstufen umfassen bzw. jeder Waschschritt kann einstufig oder mehrstufig, insbesondere einstufig, zweistufig oder dreistufig, durchgeführt werden (wobei die Wasch- und/oder Extraktionsstufen im Querstrom und/oder im Gegenstrom oder einer Kombination hiervon durchgeführt werden können).

Dabei kann in Verfahrensschritt (a) die Wäsche üblicherweise mit einem wässrig basierten Waschmedium erfolgen. Das wässrig basierte Waschmedium kann neutral, sauer oder basisch (alkalisch) eingestellt sein.

Bei dem erfindungsgemäßen Verfahren kann in Verfahrensschritt (a) als Waschmedium eine Waschsäure, eine wässrige Base oder Wasser eingesetzt werden.

Gemäß einer bevorzugten Ausführungsform der vorliegenden Erfindung kann bei dem Verfahren in Verfahrensschritt (a) die Wäsche dreischrittig und/oder in drei Waschschritten durchgeführt werden. Dabei kann die dreischrittige Wäsche bzw. die Wäsche mit drei Waschschritten einen ersten sauren Waschschritt ("saurer Waschschritt" bzw. "saure Wäsche"), einen nachfolgenden zweiten basischen (alkalischen) Waschschritt ("basischer oder alkalischer Waschschritt" bzw. ("basische oder alkalische Wäsche") und einen abschließenden dritten neutralen Waschschritt ("Neutralwaschschritt" bzw. "Neutralwäsche") umfassen. Diesbezüglich kann auf den zuvor geschilderten Stand der Technik bezüglich der Wäsche von nitrierten Rohprodukten verwiesen werden.

Gemäß einer besonderen Ausführungsform des erfindungsgemäßen Verfahrens kann in Verfahrensschritt (a) die Wäsche (im Fall mehrerer Waschschritte in jedem Waschschritt) im Querstrom oder im Gegenstrom oder aber in einer Kombination von Quer- und Gegenstrom durchgeführt werden. Dabei kann das Waschmedium vorteilhafterweise rezykliert werden.

Am Ende von Verfahrensschritt (a) erfolgt die Abtrennung des verbrauchten Waschmediums von den mit dem Waschmedium gewaschenen Dinitrotoluolen. Im allgemeinen wird in Verfahrensschritt (a) die Abtrennung des Waschmediums mittels einer Scheidevorrichtung (Scheider), insbesondere einer statischen Scheidevorrichtung, oder mittels eines Zentrifugalseparators durchgeführt. Alternativ kann die Phasentrennung auch mittels anderer, die Phasentrennung unterstützender Einrichtungen bzw. Apparaturen (z. B. Koaleszer etc.) erfolgen.

Denn die nach einer Wäsche mit Wasser anfallenden gewaschenen DNTs sind nach Abtrennung des Waschmediums stets mit Wasser gesättigt und enthalten noch Spuren an nichtgelöstem Wasser in Form einer Mikroemulsion. Die nach einer Wäsche nach statischer Scheidung erhaltenen DNTs sind deshalb meist mehr oder weniger trüb. Um den Gehalt an Wasser in den nachfolgend mittels Strippung zu behandelnden DNTs zu minimieren und damit die Strippung zu entlasten, kann - wie zuvor bereits ausgeführt - daher nach der Wäsche und vor Einspeisung der DNTs in die Strippeinrichtung der Gehalt an Wasser in den DNTs auf das in den DNTs gelöste Wasser reduziert werden, insbesondere mittels eines Zentrifugalseparators etc.

Geeignete Verfahren und Vorrichtungen (Anlagen) zur Durchführung der in Verfahrensschritt (a) durchgeführten Wäsche sind aus dem Stand der Technik grundsätzlich als solche bekannt und beispielsweise (und nicht beschränkend) in den auf die Anmelderin selbst zurückgehenden Druckschriften DE 195 12 114 A1 mit den Patentfamilienäquivalenten EP 0 736 514 A1 und US 5 756 867 A; DE 10 2005 050 106 A1 mit den Patentfamilienäquivalenten EP 1 780 195 A1 und US 2007/088183 A1; sowie DE 10 2012 009 787 A1 mit den Patentfamilienäquivalenten WO 2012/156095 A1 und CA 2 835 121 A1 beschrieben.

Was den sich Verfahrensschritt (a) anschließenden Verfahrensschritt (b) des erfindungsgemäßen Verfahrens anbelangt, so wird im Rahmen dieses Verfahrensschritts eine Strippgasbehandlung vorgenommen, d. h. die aus Verfahrensschritt (a) resultierenden gewaschenen Dinitrotoluole werden einer Strippung mit mindestens einem Gas (Strippgas) unterzogen. Wie zuvor geschildert, können auf diese Weise die nach der Wäsche noch vorhandenen, zum Teil schwer entfernbaren, insbesondere flüchtigen Verunreinigungen effizient und wirksam entfernt werden.

Bei der Strippung (synonym auch als Strippgasbehandlung, Stripping, Strippen etc. bezeichnet) handelt es sich um ein physikalisches Trennverfahren, bei welchem im Allgemeinen selektiv Stoffe (hier: Verunreinigungen) aus einer flüssigen Phase (hier: DNTs) durch Desorptionsvorgänge, insbesondere unter Ausnutzung des sogenannten Henryschen Gesetzes, in die Gasphase überführt werden. Dazu wird im Allgemeinen die Flüssigphase im Gegen- und/oder Querstromprinzip, vorzugsweise im Gegenstromprinzip, mit einem Gas (Strippgas) in Kontakt gebracht.

Bei der Strippung, einer Form der Desorption, werden also die zu entfernenden Verunreinigungen physikalisch aus einer flüssigen Phase (DNT-Phase) in die Gasphase überführt, und zwar im Allgemeinen dadurch, dass die Flüssigphase im Gegen- und/oder Querstrom mit einem deutlich größeren Volumenstrom an Gas (Strippgas) in Kontakt.gebracht wird.

Die treibende Kraft hinter dem Strippungsprozess ist, dass der Dampfdruck der aus der Flüssigkeit zu entfernenden Stoffe bzw. Verunreinigungen in der Flüssigkeit größer als in dem Gas (Strippgas) ist und daher ein Übertritt von der Flüssigkeit in das Gas (Strippgas) erfolgt.

Wie nachfolgend geschildert, wird die technische Durchführung der Strippung im Allgemeinen in geeigneten Strippvorrichtungen, wie z. B. Strippkolonnen, ausgeführt, wobei die Strippgasbehandlung durch Anpassung der apparativen Gegebenheiten sowie der Strippungsbedingungen an die zu behandelnden verunreinigten Rohprodukte zielgerichtet eingestellt und/oder sozusagen maßgeschneidert werden kann. Für weitergehende diesbezügliche Einzelheiten kann - in nichtbeschränkender Weise - beispielsweise verwiesen werden auf RÖMPP Chemielexikon, 10. Auflage, Georg-Thieme-Verlag Stuttgart/New York, 1996-1999, Band 5, Seite 4281, Stichwort: "Strippen" und Band 2, Seite 1470, Stichwort: "Gasreinigung", und auf Ullmanns Enzyklopädie der technischen Chemie, 4. Aufl., Band 2, Seiten 575 ff., sowie auf die dort jeweils referierte Literatur, wobei alle vorgenannten Literaturstellen hiermit vollumfänglich durch Bezugnahme eingeschlossen sind.

Wie zuvor dargelegt, wird die Strippung bzw. Strippgasbehandlung in hierfür geeigneten Strippvorrichtungen (Strippungsvorrichtungen) durchgeführt. Für diesen Zweck eignen sich insbesondere alle Vorrichtungen, welche es gestatten, eine große Austauschfläche zwischen Strippgas einerseits und (unter Strippbedingungen flüssigen) DNTs andererseits zu erzeugen.

Insbesondere kann in Verfahrensschritt (b) die Strippung in einer Strippungsvorrichtung durchgeführt werden, wobei als Strippungsvorrichtung eine Strippkolonne, insbesondere eine ein- oder mehrstufige Strippkolonne, bevorzugt eine Füllkörper-, Siebboden-, Glockenboden- oder Fallfilmkolonne oder Kombinationen hiervon, oder aber ein Dünnschichtverdampfer, ein Gas/Flüssig-Reaktor oder eine Horizontalstrippvorrichtung eingesetzt werden kann.

Grundsätzlich kann in Verfahrensschritt (b) die Strippung im Quer- und/oder Gegenstrom, bevorzugt im Gegenstrom, erfolgen bzw. kann in Verfahrensschritt (b) das Strippgas im Quer- und/oder Gegenstrom, bevorzugt im Gegenstrom, geführt werden.

Gemäß einer besonderen Ausführungsform der vorliegenden Erfindung kann in Verfahrensschritt (b) die Strippung mehrstufig erfolgen. Dabei kann in den verschiedenen Strippungsstufen die Strippung jeweils im Quer- oder Gegenstrom, bevorzugt im Gegenstrom, erfolgen bzw. kann in den verschiedenen Strippungsstufen das Strippgas jeweils im Quer- oder Gegenstrom, bevorzugt im Gegenstrom, geführt werden.

Um eine effiziente Durchführung der Strippgasbehandlung in Verfahrensschritt (b) zu ermöglichen, wird in Verfahrensschritt (b) des erfindungsgemäßen Verfahrens die Strippung bei Temperaturen oberhalb der Schmelztemperatur der Dinitrotoluole, insbesondere bei einer Temperatur von mindestens 56 °C, vorzugsweise bei einer Temperatur von mindestens 60 °C, bevorzugt bei einer Temperatur von mindestens 65 °C, durchgeführt. Bevorzugterweise wird in Verfahrensschritt (b) die Strippung in einem Temperaturbereich von 56 °C bis 130 °C, insbesondere in einem Temperaturbereich von 65 °C bis 105 °C, vorzugsweise in einem Temperaturbereich von 70 °C bis 95 °C, durchgeführt.

Um zu gewährleisten, dass die Strippung bei Temperaturen oberhalb der Schmelztemperatur der Dinitrotoluole erfolgt, ist es empfehlenswert, wenn in Verfahrensschritt (b) das Strippgas mit einer Strippgastemperatur im Bereich von 65 °C bis 140 °C, insbesondere im Bereich von 70 °C bis 120 °C, vorzugsweise im Bereich von 70 °C bis 95 °C, eingesetzt wird bzw. mit den Dinitrotoluolen in Kontakt gebracht wird. Zu diesem Zweck sollte in Verfahrensschritt (b) das Strippgas bei Einspeisung in die Strippvorrichtung eine Temperatur im Bereich von 65 °C bis 140 °C, insbesondere im Bereich von 70 °C bis 120 °C, vorzugsweise im Bereich von 70 °C bis 95 °C, aufweisen.

Die obere Temperatur, welche bei der Strippung der DNTs nicht überschritten werde sollte, ergibt sich insbesondere aus sicherheitstechnische Überlegungen. Da die DNTs bei der Strippung als Schmelze vorliegen sollten, ist empfehlenswert, eine sichere und effektive Behandlung der DNTs mit einem Strippgas in einem Temperaturbereich für die zu behandelnden DNTs im Bereich von 56 °C bis 130 °C, bevorzugt im Bereich von 65 °C bis 105 °C, besonders bevorzugt im Bereich von 70 °C bis 95 °C, durchzuführen. Die untere Temperaturgrenze für die Strippung wird insbesondere durch den Schmelzpunkt der DNTs vorgegeben und liegt für technische Isomerengemische, insbesondere in Abhängigkeit vom Wassergehalt in den DNTs, bevorzugt im Bereich von 55 °C bis 57 °C. Die Temperatur der DNT-Schmelze nach der Strippung sollte dabei vorzugsweise nicht unter 60 °C fallen und bevorzugt zwischen 65 °C bis 85 °C liegen, um eine Abscheidung von DNT-Kristallen (z. B. an den Apparaturen bzw. an deren Wandungen und Rohrleitungen) zuverlässig zu verhindern. Dementsprechend sollte sich die Temperatur des Strippgases in einem Bereich von 65 °C bis 140 °C, bevorzugt im Bereich von 70 °C bis 120 °C, besonders bevorzugt im Bereich von 70 °C bis 95 °C, bewegen.

Im Allgemeinen wird im Rahmen des erfindungsgemäßen Verfahrens derart vorgegangen, dass in Verfahrensschritt (b), vorzugsweise während des gesamten Verfahrens, die Dinitrotoluole im flüssigen Aggregatzustand bzw. oberhalb ihrer Schmelztemperatur vorliegen, bzw. derart, dass in Verfahrensschritt (b), vorzugsweise während des gesamten Verfahrens, die Dinitrotoluole eine Temperatur von mindestens 60 °C aufweisen und bevorzugt eine Temperatur im Bereich von 56 °C bis 130 °C, insbesondere im Bereich von 60 °C bis 105 °C, vorzugsweise im Bereich von 65 °C bis 95 °C, besonders bevorzugt im Bereich von 65 °C bis 85 °C, aufweisen.

Um eine ungewollte und gefährliche Erwärmung der DNTs über die aus sicherheitstechnischen Gründen festgelegte Temperaturobergrenze zu vermeiden, werden die DNTs in einer DNT-Produktionsanlage vorzugsweise nicht mit Standardpumpen transportiert, sondern nur mit zum Fördern von DNTs geeigneten Fördervorrichtungen, wie z. B. Zentrifugalseparatoren, welche z. B. auch bei der Phasentrennung von DNTs eingesetzt werden können, Injektoren etc., oder aber alternativ gravimetrisch.

Grundsätzlich kann die Strippgasbehandlung bzw. Strippung nicht nur in einem weiten Temperaturbereich, sondern auch in einem weiten Druckbereich durchgeführt werden, jedoch stets mit der Maßgabe, dass bei der Strippung bzw. Strippgasbehandlung die zu behandelnden DNTs im flüssigen Aggregatzustand vorliegen sollten.

Grundsätzlich kann in Verfahrensschritt (b) die Strippung bei Atmosphärendruck (Normaldruck; 1,01325 bar) oder aber im Vakuum (Unterdruck) oder aber bei Überdruck, vorzugsweise bei Atmosphärendruck, durchgeführt werden. Im Allgemeinen kann in Verfahrensschritt (b) die Strippung bei einem Absolutdruck im Bereich von 0,2 bar bis 3 bar, insbesondere im Bereich von 0,3 bar bis 1,2 bar, vorzugsweise im Bereich von 0,4 bar bis 1,1 bar, besonders bevorzugt bei etwa 1 bar, durchgeführt werden.

Die Behandlung mit dem Strippgas erfolgt bevorzugt bei Atmosphärendruck (Normaldruck). Aber zum Zwecke der Optimierung der Strippgasmenge ist grundsätzlich auch eine Strippung im Vakuum oder bei insbesondere leichtem Überdruck möglich.

Was das bei der Strippung eingesetzte Gas (Strippgas) anbelangt, so können hier unterschiedlichste Gase zum Einsatz kommen. Erfindungsgemäß wird in Verfahrensschritt (b) als Strippgas ein gegenüber Dinitrotoluolen nichtreaktives bzw. gegenüber Dinitrotoluolen inertes Gas eingesetzt. Dabei wird das Strippgas ausgewählt aus der Gruppe von Stickstoff, Sauerstoff, Edelgasen (vorzugsweise Helium und Argon), Wasserstoff, Kohlenstoffoxiden (z. B. Kohlenmonoxid und Kohlendioxid) und deren Mischungen (insbesondere Luft als Stickstoff/SauerstoffGemisch), vorzugsweise aus der Gruppe von Stickstoff, Sauerstoff, Edelgasen und deren Mischungen, besonders bevorzugt aus der Gruppe von Stickstoff, Sauerstoff und deren Mischungen. Ganz besonders bevorzugt wird als Strippgas Stickstoff oder Luft eingesetzt, da dies die kostengünstigsten und technisch am besten verfügbaren Strippgase darstellen.

Die konkrete Auswahl des Strippgases hängt insbesondere auch davon ab, welche Verunreinigungen bzw. Stoffe aus den zu behandelnden DNTs entfernt werden sollen: Soll beispielsweise ein kohlenstoffoxidfreies Rein-DNT bereitgestellt werden, so wird der Fachmann selbstverständlich nur auf solche der vorgenannten Strippgase zurückgreifen, welche frei von Kohlenstoffoxiden ausgebildet sind.

Gemäß einer typischen Ausführungsform des erfindungsgemäßen Verfahrens können als Strippgas für das Strippen der gewaschenen DNTs insbesondere alle Inertgase (d. h. alle gegenüber den gewaschenen DNTs unter Strippgasbehandlungsbedingungen nichtreaktiven Gase), insbesondere sofern sie preisgünstig zur Verfügung stehen, zum Einsatz kommen, wie z. B. Luft, Stickstoff, Edelgase, CO₂, CO, N₂O, Wasserstoff etc. Bevorzugt werden Luft oder Stickstoff eingesetzt, aber auch andere Strippgase können, insbesondere unter Beachtung der einzuhaltenden Strippgasbehandlungsparameter, wie vor allem Druck und Temperatur, eingesetzt werden, wobei das DNT jeweils mit dem Strippgas bis zu dessen Löslichkeitsgrenze unter Beachtung des Henryschen Gesetzes beladen wird.

Im Allgemeinen sollte das Strippgas zumindest im Wesentlichen frei von Wasser und/oder Wasserdampf, insbesondere zumindest im Wesentlichen frei von Wasserdampf, ausgebildet sein. Im anderen Worten sollte im Allgemeinen in Verfahrensschritt (b) das Strippgas kein Wasser und/oder keinen Wasserdampf, insbesondere keinen Wasserdampf, enthalten. Dies bedeutet, dass in Verfahrensschritt (b) die Strippung in Abwesenheit von Wasser und/oder Wasserdampf, insbesondere in Abwesenheit von Wasserdampf, durchgeführt wird bzw. in Verfahrensschritt (b) als Strippgas kein Wasserdampf eingesetzt wird.

Dennoch ist grundsätzlich aber auch eine Beladung des Strippgases mit einer (relativen) Luft- bzw. Restfeuchte von bis zu 100 Gew.-%, insbesondere bis zu 90 Gew.-%, bevorzugt bis zu 70 Gew.-% und weniger, insbesondere bezogen auf Strippgasbehandlungstemperatur, vorzugsweise bezogen auf eine Gastemperatur von 20 °C bis 30 °C, möglich.

Denn, wie die Anmelderin herausgefunden hat, ist die Verwendung von Wasserdampf als Strippgas aus verschiedenen Gründen in Bezug auf das erfindungsgemäße Verfahren abträglich: Zum einen würde durch die Verwendung von Wasserdampf als Strippgas Wasser in die zu behandelnden DNTs eingetragen werden. Aber auch aus sicherheitstechnischen Gründen ist Wasserdampf für die Strippung im Rahmen des erfindungsgemäßen Verfahrens nicht bzw. nicht gut geeignet. Ein weiterer Nachteil der Strippung mit Wasserdampf ist, dass - neben dem Strippgas mit den abgetrennten Verunreinigungen - stets auch ein DNT/ Wasser-Gemisch im Kolonnensumpf der Strippkolonne anfällt, welches wiederum abgetrennt werden muss, so dass neben zusätzlichem technischem Aufwand ein zusätzlicher Abfallstrom anfallen würde. Daher sollte Wasserdampf als Strippgas vermieden werden (auch wenn es grundsätzlich in technischer Hinsicht einsetzbar ist bzw. in seiner Verwendung zur Durchführung eines entsprechenden Verfahrens grundsätzlich realisierbar ist).

Wasserdampf ist somit als Strippgas - insbesondere unter Beachtung der bevorzugten Temperaturbereiche für die Strippgasbehandlung, welche aus sicherheitstechnischen Gründen zu beachten sind - nicht bzw. nicht in besonderem Maße geeignet: Eine Strippung mit Wasserdampf im bevorzugten Temperaturbereich von 105 °C und kleiner ist für eine mit Strippgas zu behandelnde DNT-Schmelze im Vakuum in technischer Hinsicht sehr aufwendig. Ein weiterer Nachteil der Strippung einer DNT-Schmelze mit Sattdampf ist, dass - wie zuvor geschildert - neben dem Strippgas mit den abgetrennten leichtflüchtigen Verunreinigungen stets auch eine DNT/Wasser-Gemisch im Kolonnensumpf der Strippkolonne anfällt, welches abgetrennt werden muss; in dieses im Kolonnensumpf anfallende Wasser werden die in den zu strippenden DNTs noch vorliegenden starken und mittelstarken Säuren, wie Salpetersäure, Trinitrokresole und Nitrobenzoesäuren, extrahiert. Es fällt folglich ein zusätzlicher Abfallstrom an, welcher verfahrenstechnisch aufwendig zu handhaben ist. Folglich ist erfindungsgemäß das einzusetzen Strippgas frei von Wasserdampf ausgebildet.

Bevorzugt wird das Strippgas in trockener bzw. getrockneter Form eingesetzt (d. h. zumindest im Wesentlichen frei von Wasser), insbesondere da durch die Strippgasbehandlung auch der Restwassergehalt im zu strippenden DNT reduziert werden soll. Grundsätzlich ist aber auch eine Beladung des Strippgases mit einer (relativen) Luft- bzw. Restfeuchte von bis zu 100 Gew.-%, insbesondere bis zu 90 Gew.-%, bevorzugt bis zu 70 Gew.-% und weniger, insbesondere bezogen auf Strippgasbehandlungstemperatur, vorzugsweise bezogen auf eine Gastemperatur von 20 °C bis 30 °C, möglich.

Die in Verfahrensschritt (b) eingesetzte Menge an Strippgas kann in weiten Grenzen variieren.

Insbesondere wird in Verfahrensschritt (b) die eingesetzte Menge an Strippgas in Abhängigkeit von den Bedingungen der Strippung, insbesondere Temperatur und/oder Druck und/oder Dauer der Strippung und/oder Art des Strippgases, und/oder in Abhängigkeit von dem Gehalt und/oder Art an Verunreinigungen in den in Verfahrensschritt (b) zu behandelnden Dinitrotoluolen und/oder in Abhängigkeit von dem angestrebten Restgehalt an Verunreinigungen in den nach Verfahrensschritt (b) resultierenden Dinitrotoluolen gewählt und/oder eingestellt.

Gemäß einer typischen Ausführungsform der vorliegenden Erfindung kann die Menge an einzusetzendem Strippgas, beispielsweise berechnet als Gewichtsverhältnis von Strippgas zu DNTs, in Abhängigkeit von Temperatur und Druck, bei welchen die Strippung erfolgt, und in Abhängigkeit von dem gewünschten Restgehalt der einzelnen Verunreinigungen gewählt und/oder eingestellt werden.

Gemäß einer besonderen Ausführungsform beträgt in Verfahrensschritt (b) die eingesetzte Menge an Strippgas, bezogen auf 1 Tonne zu behandelnder Dinitrotoluole und berechnet als Volumen an Strippgas in Normkubikmetern unter Standardbedingungen (Druck von 1,01325 bar; Luftfeuchtigkeit von 0 %, d. h. trockenes Strippgas; Temperatur von 0 °C [Normbedingungen nach DIN 1343, STPD]), 0,1 bis 1.000 Normkubikmeter Strippgas, insbesondere 0,5 bis 500 Normkubikmeter, vorzugsweise 1 bis 200 Normkubikmeter, besonders bevorzugt 2 bis 120 Normkubikmeter, ganz besonders bevorzugt 5 bis 80 Normkubikmeter, noch mehr bevorzugt 10 bis 60 Normkubikmeter.

Der Normkubikmeter (synonym auch als Normalkubikmeter bezeichnet) ist eine in der Verfahrenstechnik und in der Gastechnik verwendete Maßeinheit für eine Gasmenge. Sie beschreibt diejenige Gasmenge, welche unter festgelegten Bedingungen (Temperatur, Druck, Luftfeuchtigkeit) ein Gasvolumen von einem Kubikmeter einnehmen würde. Die Einheit Normkubikmeter bzw. die Umrechnung in Normkubikmeter wird verwendet, um Mengenangaben im Zusammenhang mit Gasen auf eine vergleichbare Maßeinheit in einem willkürlich festgelegten, aber genau definierten Zustand bezüglich Temperatur, Druck und Feuchte zu beziehen. Im Rahmen der vorliegenden Erfindung wird die Maßeinheit des Normkubikmeters in Bezug auf Standardbedingungen bzw. Normbedingungen verwendet (Druck von 1,01325 bar; Luftfeuchtigkeit von 0 %, d. h. trockenes Strippgas; Temperatur von 0 °C [Normbedingungen nach DIN 1343, STPD]).

Ein Normkubikmeter ist im Rahmen der vorliegenden Erfindung also diejenige Gasmenge, die bei einem Druck von 1,01325 bar und einer Luftfeuchtigkeit von 0 Gew.-% (trockenes Strippgas) und einer Temperatur von 273,15 K (0 °C) ein Volumen von einem Kubikmeter Gas hat.

Ein Normkubikmeter (ideales Gas) enthält eine konstante Stoffmenge von 44,615 Mol an Gasmolekülen (unabhängig von der Art des Gases).

Ein Normkubikmeter folgender Gasarten enthalten die nachfolgend genannten Massen an Gasen:

| | |
|---|---|
| Luft: | 1,293 kg |
| Stickstoff: | 1,250 kg |
| Wasserstoff: | 0,0899 kg |

Gemäß einer besonderen Ausführungsform kann in Verfahrensschritt (b) die eingesetzte Menge an Strippgas, bezogen auf 1 Tonne zu behandelnder Dinitrotoluole und berechnet als Menge an Stickstoff als Strippgas unter Standardbedingungen (Druck von 1,01325 bar; Luftfeuchtigkeit von 0 %, d. h. trockenes Strippgas; Temperatur von 0 °C [Normbedingungen nach DIN 1343, STPD]), 0,5 bis 500 kg, insbesondere 1 bis 250 kg, vorzugsweise 1 bis 140 kg, besonders bevorzugt 5 bis 50 kg, noch mehr bevorzugt 10 bis 40 kg, betragen. Sofern mit anderen Inertgasen gearbeitet wird, sind die für Stickstoff als Strippgas angegebenen Werte entsprechend anzupassen bzw. umzurechnen.

Im Allgemeinen erfolgt in Verfahrensschritt (b) gleichermaßen eine Trocknung und/oder eine Reduktion des Wassergehalts der behandelten Dinitrotoluole. Insbesondere beträgt in diesem Zusammenhang der Wassergehalt in den nach Verfahrensschritt (b) resultierenden Dinitrotoluolen höchstens 1 Gew.-%, insbesondere höchstens 0,8 Gew.-%, vorzugsweise höchstens 0,5 Gew.-%, besonders bevorzugt höchstens 0,4 Gew.-%, ganz besonders bevorzugt höchstens 0,3 Gew.-%, noch mehr bevorzugt höchstens 0,2 Gew.-%, jeweils bezogen auf die Dinitrotoluole.

Im Allgemeinen wird in Verfahrensschritt (b) der Gehalt an Wasser in den resultierenden Dinitrotoluolen derart abgesenkt, dass bei Abkühlung der flüssigen Dinitrotoluole bis zu deren Erstarrungspunkt kein Wasser mehr abgeschieden wird.

Beim Behandeln eines in Verfahrensschritt (a) gewaschenen DNT mit Inertgasen, wie z. B. Luft oder Stickstoff, in einer Strippeinrichtung werden also nicht nur niedrigsiedende aliphatische und aromatische Kohlenwasserstoffe, Blausäure, Ammoniak, Salpetersäure etc. und die bereits bei Raumtemperatur gasförmigen Verunreinigungen im DNT, wie z. B. CO, N₂O, CO₂, NO, NO₂ etc., und die aus NOx in Gegenwart von Wasser neu gebildete Salpetersäure entfernt, sondern auch das im DNT gelöste und gegebenenfalls suspendierte Wasser. Der Anteil des noch gelösten Wassers im DNT ist nach Behandlung mit dem Strippgas derart gering bzw. sollte nach Behandlung mit dem Strippgas derart reduziert sein, dass bei Abkühlung des flüssigen DNT bis zu seinem Schmelzpunkt kein Wasser mehr abgeschieden werden kann und damit der "Taupunkt" nicht mehr erreicht wird. Damit wird die Bildung von Spuren an Wasser in einem DNT-Zwischenlager, welches aufgrund der stets vorhandenen Restacidität im gewaschenen und mit Strippgas behandelten DNT hochkorrosiv ist, sicher vermieden.

Gemäß einer besonderen Ausführungsform kann in Verfahrensschritt (b) die Strippung nach einer vorangehenden sauren, basischen oder neutralen Wäsche gemäß Verfahrensschritt (a) erfolgen. Dies bedeutet, dass im Rahmen des erfindungsgemäßen Verfahrens die vorgeschaltete Wäsche gemäß Verfahrensschritt (a) ein-, zwei- oder Dreischritte ausgestaltet sein kann bzw. ein, zwei oder drei Waschschritte umfassen kann. Beispielsweise kann die Behandlung mit dem Strippgas nach einem ersten Waschschritt (saure Wäsche) und/oder nach einem zweiten Waschschritt (alkalische Wäsche) und/oder nach einem dritten Waschschritt (neutrale Wäsche) erfolgen; dies wird im Zusammenhang mit den nachfolgend diskutierten Figurendarstellungen und den Ausführungsbeispielen noch im Detail erläutert. Aber auch nach einzelnen Extraktionsstufen bzw. einzelnen Waschstufen der verschiedenen Waschschritte ist eine Behandlung mit einem Strippgas möglich.

Gemäß einer besonderen Ausführungsform der vorliegenden Erfindung kann nach Durchführung von Verfahrensschritt (b) das mit den aus den Dinitrotoluolen entfernten Verunreinigungen beladene Strippgas einer Abgasbehandlung, insbesondere einer thermischen Abgasbehandlung, unterworfen werden.

Gemäß einer hierzu alternativen Ausführungsform kann nach Durchführung von Verfahrensschritt (b) das mit den aus den Dinitrotoluolen entfernten Verunreinigungen beladene Strippgas einer Behandlung zur Entfernung der Verunreinigungen aus dem beladenen Strippgas, vorzugsweise mittels Kondensation und/oder Wäsche mit einem vorzugsweise wässrigen Waschmedium, unterworden werden, wobei sich gegebenenfalls eine Rezyklierung des aufgereinigten Strippgases in Verfahrensschritt (b) anschließen kann.

Beispielsweise kann das Strippgas nach Abtrennung der leicht kondensierbaren bzw. leicht auswaschbaren Verunreinigungen, wie z. B. Salpetersäure, Blausäure etc., und der bei Raumtemperatur flüssigen aliphatischen und aromatischen Kohlenwasserstoffe im Kreise gefahren werden, wobei vorteilhafterweise ein Teilstrom des Strippgases, welcher mit nichtkondensierbaren oder nichtauswaschbaren Inertstoffen, wie CO, N₂O etc., beladen ist, ausgeschleust und einer Abgasbehandlung, z. B. einer thermischen Abgasbehandlung, zugeführt wird, um eine Anreicherung dieser Inertstoffe im Strippgasstrom zu vermeiden.

Alle mit dem Strippgas aus den zuvor gewaschenen DNTs entfernten Verunreinigungen sowie auch das abgetrennte Wasser befinden sich im Abgasstrom. Dieser Abgasstrom kann beispielsweise ohne Zwischenkondensation einer thermischen Abgabehandlung zugeführt werden, wenn dafür gesorgt wird, dass das aus den DNTs entfernte Wasser nicht kondensiert. Aber auch eine Zwischenkondensation oder eine zusätzliche Wäsche mit einem Waschmedium, wie Wasser oder einer Waschsäure, ist möglich, wenn die in Wasser gut löslichen Säuren, wie Salpetersäure, NOx, Blausäure etc., und die Kohlenwasserstoffe vom Abgas abgetrennt werden sollen, damit die thermische Abgasbehandlung entlastet wird.

Das bei einer Zwischenkondensation bzw. bei einer zusätzlichen Behandlung des Abgases mit einem Waschmedium anfallende Abwasser, welches vor allem Salpetersäure, Blausäure und andere gut wasserlösliche Verunreinigungen sowie DNT und Spuren von abgetrennten Kohlenwasserstoffen enthält, kann - allein oder gemeinsam mit dem Brüdenkondensat aus der Aufkonzentrierung der Waschsäure aus dem ersten Waschschritt (d. h. saure Wäsche) von Verfahrensschritt a), wie z. B. in der EP 0 736 514 A1 bzw. in der EP 1 780 198 A1 beschrieben - vom gelösten und suspendierten DNT durch Extraktion mit dem zu nitrierenden Aromaten (d. h. bei DNT mit Toluol) befreit werden. Die Blausäure kann im extrahierten Abwasser direkt, z. B. durch Behandlung mit Oxidationsmitteln, oder durch Strippen mit Dampf zusammen mit dem Extraktionsmittel entfernt werden. Nach Neutralisation kann dieses vereinigte Abwasser dann entweder in eine biologische Nachbehandlung oder direkt in einen Vorfluter abgegeben werden.

Die im Strippkondensat aus den vereinigten Abwässern angereicherte Blausäure muss, bevor dieses Kondensat ebenfalls in eine Abwasserbehandlung abgegeben wird, durch eine Behandlung mit Oxidationsmitteln oder andere Maßnahmen zerstört oder gebunden werden. Ein Teilstrom der abgetrennten leichtflüchtigen Kohlenwasserstoffe, welche noch Spuren an MNT und DNT enthalten, können in die Wäsche zurückgeführt werden.

Das Abgas wird vor Abgabe in die Umgebung typischerweise durch eine Nachverbrennung aufoxidiert und abgegeben.

Wie zuvor bereits ausgeführt, gelingt es mit dem erfindungsgemäßen Verfahren, auf technisch einfache und sichere Weise zumindest im Wesentlichen sämtliche bzw. nahezu sämtliche Verunreinigungen aus den Rohdinitrotoluolen, wie sie bei einer z. B. adiabatisch oder isotherm durchgeführten Nitrierung von Toluol in Gegenwart eines Salpetersäure/Schwefelsäure-Nitriersäuregemisches anfallen, effizient zu entfernen, und zwar auch solche Verunreinigungen, insbesondere auch flüchtige Verunreinigungen (aber auch andere Verunreinigungen, wie nichtacide oder schwachacide Verunreinigungen), welche sich mit herkömmlichen Aufreinigungsverfahren für Rohdinitrotoluole nicht bzw. nicht ohne Weiteres entfernen lassen. Überraschenderweise lassen sich insbesondere durch die nachgeschaltete Strippgasbehandlung zudem wirksam auch Spurenverunreinigungen, welche aber die nachfolgende Weiterverwendung oder Weiterverarbeitung der Dinitrotoluole massiv beeinträchtigen können (z. B. als Katalysatorgifte eine katalytische Hydrierung der Dinitrotoluole beeinträchtigen können), wirksam entfernen.

Die mit Hilfe des erfindungsgemäßen Verfahrens aus den Rohdinitrotoluolen zu entfernenden Verunreinigungen können insbesondere ausgewählt sein aus der Gruppe von Mineralsäuren (insbesondere Schwefelsäure, Salpetersäure und Salpetrige Säure), nichtumgesetzten und/oder teilumgesetzen Edukten, Stickstoffoxiden, Kohlenstoffoxiden und nitrierten und nichtnitrierten Reaktionsnebenprodukten sowie Abbau- und Oxidationsprodukten der aus der Nitrierung stammenden Produkte und Nebenprodukte (wie z. B. Blausäure etc.).

Insbesondere können die mit Hilfe des erfindungsgemäßen Verfahrens aus den Rohdinitrotoluolen zu entfernenden Verunreinigungen ausgewählt sein aus der Gruppe von (i) Mineralsäuren, insbesondere Schwefelsäure, Salpetersäure und Salpetrige Säure; (ii) organischen Säuren, insbesondere Ameisensäure, Essigsäure, Oxalsäure, Nitrobenzoesäuren, wie Mono- und Dinitrobenzoesäuren, Pikrinsäure, Blausäure oder dergleichen; (iii) Nitroaromaten, insbesondere Nitrophenolen, wie Mono-, Di- und Trinitrophenolen, und Nitrokresolen, wie Mono-, Di- und Trinitrokresolen; (iv) Stickstoffoxiden (Stickoxiden), insbesondere Stickstoffmonoxid (NO), Stickstoffdioxid (NO₂) und Distickstoffoxid (N₂O); (v) Kohlenstoffoxiden, insbesondere Kohlenmonoxid (CO) und Kohlendioxid (CO₂); (vi) Kohlenwasserstoffen, insbesondere aliphatischen und cycloaliphatischen Kohlenwasserstoffen; (vii) nichtumgesetzten oder teilumgesetzten Edukten, insbesondere Toluol.

Wie zuvor ausgeführt, lassen sich in nicht erwartbarer Weise durch das erfindungsgemäße Verfahren aus den aufzureinigenenden Rohdinitrotoluolen insbesondere auch (aber eben nicht nur) flüchtige Verunreinigungen entfernen. Hierunter werden im Rahmen der vorliegenden Erfindung insbesondere (aber nicht beschränkend) solche Verbindungen verstanden, welche eine höhere Flüchtigkeit, insbesondere einen höheren Dampfdruck und/oder einen geringeren Siedepunkt, als die aufzureinigenden Rohdinitrotoluole aufweisen. Als Bezugsgröße für die Flüchtigkeit kann beispielsweise der Dampfdruck der Dinitrotoluole unter Standardbedingungen (d. h. Druck von 1,01325 bar; Luftfeuchtigkeit von 0 % [Normbedingungen nach DIN 1343, STPD]) und/oder der Siedepunkt der Dinitrotoluole bei Standardbedingungen (d. h. Druck von 1,01325 bar; Luftfeuchtigkeit von 0 % [Normbedingungen nach DIN 1343, STPD]) herangezogen werden. Wie zuvor ausgeführt, gehören zu den flüchtigen Verbindungen, welche im Rahmen des erfindungsgemäße Verfahrens entfernt werden können, beispielsweise Stickstoffoxide (z. B. NO, NO₂, N₂O etc.), Kohlenwasserstoffe (z. B. aliphatische oder aromatische Kohlenwasserstoffe etc.), Kohlenstoffoxide (z. B. CO, CO₂ etc.), Blausäure etc.

Wie zuvor ausgeführt, ist das erfindungsgemäße Verfahren mit einer Vielzahl von Vorteilen und Besonderheiten verbunden, welche die vorliegende Erfindung gegenüber dem einschlägigen Stand der Technik abgrenzen:
Im Rahmen des erfindungsgemäßen Verfahrens werden also aus einer adiabatischen oder isothermen Dinitrierung von Toluol erhaltene rohe Dinitrotoluole, welche zunächst in einem oder mehreren insbesondere in ein bis drei Waschschritten gewaschen werden und somit nur noch Spuren an Salpetersäure, Stick(stoff)oxiden NOx und Schwefelsäure, aber immer noch beträchtliche Mengen an schlecht auswaschbaren Verunreinigungen der vorgenannten Art enthalten, mit einem Strippgas behandelt. Durch diese Behandlung der zuvor gewaschenen DNTs mit einem Strippgas werden zumindest im Wesentlichen alle bzw. nahezu alle Verunreinigungen mit einem niedrigen Siedepunkt und folglich hohen Partialdrücken im Vergleich zu den DNTs, insbesondere die in den DNTs vorliegenden, bereits bei Raumtemperatur gasförmigen Verunreinigungen, wie z. B. CO, CO₂, N₂O, NO, NO₂ etc., aber auch alle Verunreinigungen mit niedrigen Siedepunkten, wie z. B. Blausäure, Salpetersäure, aliphatische und aromatische Kohlenwasserstoffe, Wasser etc., welche mit einer Wäsche mit Wasser gar nicht nur durch eine vielstufige flüssig/flüssig-Extraktion abgetrennt werden können, entsprechend ihren Partialdrücken nach den Regeln des Henryschen Gesetzes effektiv und ohne großen technischen Aufwand vollständig bzw. gezielt bis auf die gewünschten Restgehalte entfernt.

Das erfindungsgemäße Verfahren ist besonders dann vorteilhaft, wenn im Rahmen der Wäsche gemäß Verfahrensschritt (a) bei großen Verhältnissen von zu waschenden DNTs zu frisch zugesetztem Waschmedium gearbeitet wird und/oder wenn die aus den Roh-DNTs zu entfernenden Verunreinigungen mit kleinen Extraktionskoeffizienten physikalisch nur durch eine vielstufige Wäsche aus den DNTs abtrennbar sind.

Ein weiterer Vorteil des Verfahrens besteht darin, dass es äußerst flexibel ausführbar ist, da bei einer mehrstufigen Wäsche bzw. einer Wäsche mit mehreren Waschschritten (z. B. Wäsche mit drei Waschschritten) nach jedem Waschschritt nach Phasentrennung die DNTs mit einem Strippgas behandelt werden können: Nach dem erfindungsgemäßen Verfahren lassen sich sowohl ein in drei Waschschritten gewaschenes DNT, welches immer noch Restspuren von Blausäure, Ammoniak (mit Ammoniak als Base im alkalischen bzw. zweiten Waschschritt) und vor allem größere Mengen an Neutralstoffen, wie CO, N₂O etc. und niedrig siedenden aliphatischen und aromatischen Kohlenwasserstoffen, und darüber hinaus beträchtliche Restmengen an NOx enthalten kann, als auch ein DNT aus dem ersten bzw. sauren Waschschritt (behandelt mit einer Waschsäure, wie z. B. in der EP 0 297 312 A1, der EP 0 736 514 A1 oder der EP 1 780 195 A1 beschrieben), welches zusätzlich noch Nitrophenole, Nitrokresole, Nitrobenzoesäuren neben allen Verunreinigungen mit ungünstigen Verteilungskoeffizienten, geringer Löslichkeit im Waschmedium, schwacher oder fehlender Acidität, wie z. B. CO, CO₂, Blausäure, NOx, N₂O etc., und auch noch Verunreinigungen, welche zum Teil gefährliche Katalysatorgifte darstellen, sowie niedrigsiedende aliphatische und aromatische Kohlenwasserstoffe etc. in größerer Menge enthält, von diesen Verunreinigungen in einfacher und effizienter Weise befreien. Aber auch ein DNT nach dem zweiten Waschschritt (d. h. nach der alkalischen Wäsche) kann mit einem geeigneten Strippgas effizient behandelt werden. Aber auch nach jeder Extraktionsstufe eines aus mehreren Extraktionsstufen bestehenden Waschschrittes ist eine Behandlung des nach Abtrennung des Waschmedium anfallenden DNT mit einem Strippgas möglich.

Das erfindungsgemäße Verfahren arbeitet also in technischer Hinsicht zuverlässig, sicher, effizient und flexibel und ist apparativ leicht umsetzbar, ist aber gleichzeitig auch ökonomisch in seinem Betrieb.

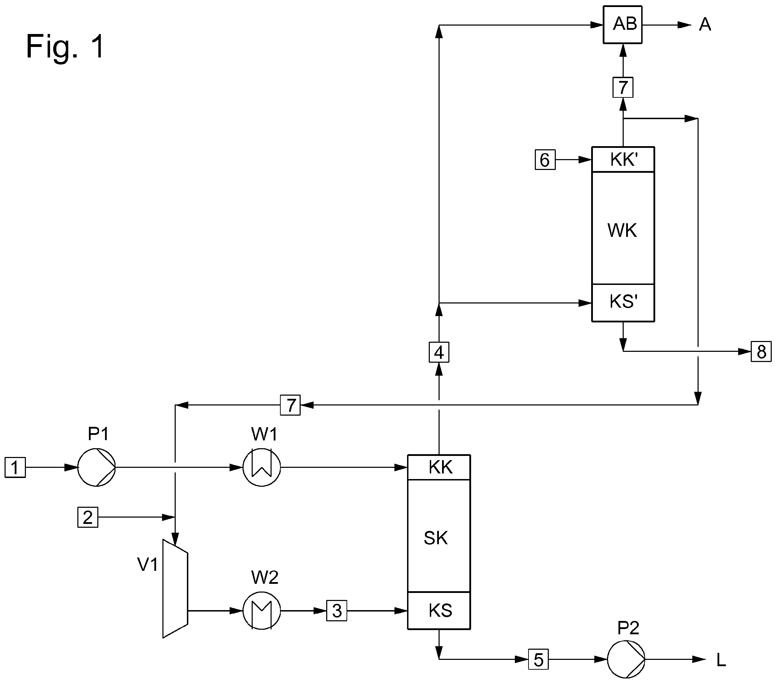

Nachfolgend werden die beiden anderen Aspekte der vorliegenden Erfindung - erfindungsgemäße Vorrichtung (Anlage) zur Aufreinigung von aus der Nitrierung von Toluolen in Gegenwart eines Salpetersäure/Schwefelsäure-Nitriersäuregemisches resultierenden Rohdinitrotoluolen einerseits (zweiter erfindungsgemäßer Aspekt) und erfindungsgemäße Produktionsanlage zur Herstellung von Dinitrotoluolen andererseits (dritter erfindungsgemäßer Aspekt) - beschrieben, wobei die nachfolgend angegebenen Bezugszeichen sich auf die anmeldungsgemäßen Figurendarstellungen gemäß Figs. 1 bis 4 beziehen, welche im Anschluss noch einmal im Detail erläutert werden.

Die vorliegende Erfindung betrifft - gemäß einem **zweiten** Aspekt der vorliegenden Erfindung - eine Vorrichtung (Anlage) zur Aufreinigung von aus der Nitrierung von Toluol in Gegenwart eines Salpetersäure/Schwefelsäure-Nitriersäuregemisches resultierenden Rohdinitrotoluolen nach Abtrennung des Nitrierendsäuregemisches (synonym auch als Nitrierendsäure bezeichnet), insbesondere zur Entfernung auch von insbesondere flüchtigen Verunreinigungen aus den Rohdinitrotoluolen, insbesondere eine Vorrichtung (Anlage) zur Durchführung eines wie zuvor beschriebenen Verfahrens gemäß der vorliegenden Erfindung,
wobei die Vorrichtung umfasst:
(a) mindestens eine Wascheinheit WE zur Wäsche der Rohdinitrotoluole R-DNT mit mindestens einem Waschmedium WM; und,
(b) stromabwärts zur Wascheinheit WE angeordnet, mindestens eine Strippeinrichtung (Strippvorrichtung) SK zur Strippung (Strippgasbehandlung) der aus der Wascheinheit WE hervorgehenden gewaschenen Dinitrotoluole W-DNT; DNT-I, DNT-II, DNT-III mit mindestens einem gegenüber Dinitrotoluolen nichtreaktiven und/oder gegenüber Dinitrotoluolen inerten Gas (Strippgas) SG, ausgewählt ist aus der Gruppe von Stickstoff, Sauerstoff, Edelgasen, Wasserstoff, Kohlenstoffoxiden und deren Mischungen, in Abwesenheit von Wasserdampf.

Gemäß einer besonderen Ausführungsform der vorliegenden Erfindung kann bei der erfindungsgemäßen Vorrichtung die Wascheinheit WE ein oder mehrere, insbesondere ein, zwei oder drei, vorzugsweise drei, Wascheinrichtungen WS-I, WS-II, WS-III zur Wäsche der Rohdinitrotoluole R-DNT, insbesondere eine Wascheinrichtung WS-I zur Durchführung einer sauren Wäsche und/oder eine Wascheinrichtung WS-II zur Durchführung einer alkalischen Wäsche und/oder eine Wascheinrichtung WS-III zur Durchführung einer neutralen Wäsche, aufweisen.

Gemäß einer bevorzugten Ausführungsform der vorliegenden Erfindung kann bei der erfindungsgemäßen Vorrichtung die Wascheinheit WE drei Wascheinrichtungen WS-I, WS-II, WS-III zur Wäsche der Rohdinitrotoluole R-DNT aufweisen, und zwar umfassend eine Wascheinrichtung WS-I zur Durchführung einer sauren Wäsche, eine zu der Wascheinrichtung WS-I zur Durchführung der sauren Wäsche stromabwärts angeordnete Wascheinrichtung WS-II zur Durchführung einer alkalischen Wäsche und eine stromabwärts zu der Wascheinrichtung WS-II zur Durchführung der alkalischen Wäsche angeordnete Wascheinrichtung WS-III zur Durchführung einer neutralen Wäsche.

Gemäß einer Ausführungsform der vorliegenden Erfindung kann bei der erfindungsgemäßen Vorrichtung die Wascheinheit WE zusätzlich mindestens eine Abtrenneinrichtung zur Abtrennung des gebrauchten und/oder mit den aus den Dinitrotoluolen entfernten Verunreinigungen beladenen Waschmediums WL von den gewaschenen Dinitrotoluolen W-DNT; DNT-I, DNT-II, DNT-III umfassen.

Gemäß einer besonderen Ausführungsform der vorliegenden Erfindung kann die Strippeinrichtung SK, insbesondere eine Strippkolonne, ein unteres Ende (Unterteil) KS, insbesondere einen Kolonnensumpf, und ein oberes Ende (Oberteil) KK, insbesondere einen Kolonnenkopf, aufweisen und eine vorzugsweise am unteren Ende KS angeordnete Zuführeinrichtung für das Strippgas SG und eine vorzugsweise am oberen Ende KK angeordnete Zuführeinrichtung für die gewaschenen Dinitrotoluole W-DNT; DNT-I, DNT-II, DNT-III umfassen.

Vorzugsweise kann die Strippeinrichtung SK als Strippkolonne mit einem Kolonnensumpf KS als unterem Ende (Unterteil) und einem Kolonnenkopf KK als oberem Ende (Oberteil) ausgebildet sein. Dabei kann die Strippkolonne eine vorzugsweise am Kolonnensumpf KS angeordnete Zuführeinrichtung für das Strippgas SG und eine vorzugsweise am Kolonnenkopf KK angeordnete Zuführeinrichtung für die gewaschenen Dinitrotoluole W-DNT; DNT-I, DNT-II, DNT-III umfassen.

Gemäß einer besonderen Ausführungsform der vorliegenden Erfindung kann die Strippeinrichtung SK derart ausgebildet ist, dass die Strippeinrichtung SK im Betriebszustand einen Gegen- und/oder Querstromfluss, vorzugsweise einen Gegenstromfluss, von gewaschenen Dinitrotoluolen W-DNT; DNT-I, DNT-II, DNT-III einerseits und Strippgas SG andererseits ermöglicht.

Bezüglich weitergehender Einzelheiten zu der erfindungsgemäßen Vorrichtung (Anlage) kann zur Vermeidung unnötiger Wiederholungen auf die obigen Ausführungen zu dem erfindungsgemäßen Verfahren verwiesen werden, welche in Bezug auf die erfindungsgemäße Vorrichtung (Anlage) entsprechend gelten.

Die vorliegende Erfindung betrifft - gemäß einem **dritten** Aspekt der vorliegenden Erfindung - eine Produktionsanlage zur Herstellung von Dinitrotoluolen, insbesondere eine Produktionsanlage zur Nitrierung (Dinitrierung) von Toluol zu Dinitrotoluolen in Gegenwart eines Salpetersäure/Schwefelsäure-Nitriersäuregemisches mit nachfolgender Aufreinigung der aus der Nitrierung hervorgehenden Rohdinitrotoluole,
wobei die Produktionsanlage umfasst:
(i) eine Nitriereinrichtung NE zur Nitrierung (Dinitrierung) von Toluol T in Gegenwart eines Salpetersäure/Schwefelsäure-Nitriersäuregemisches MS zu Dinitrotoluolen R-DNT, insbesondere mit einem oder mehreren Reaktionsbehältern (Nitrierreaktoren) zur Durchführung der Nitrierung;
(ii) gegebenenfalls, in Produktionslinie stromabwärts zur Nitriereinrichtung NE angeordnet, mindestens eine Abtrenneinrichtung, insbesondere eine Scheideeinrichtung (Scheider), zur Abtrennung des Nitrierendsäuregemisches SP von den Rohdinitrotoluolen R-DNT;
(iii) in Produktionslinie stromabwärts zur Nitriereinrichtung NE und zur gegebenenfalls vorhandenen Abtrenneinrichtung angeordnet, eine Vorrichtung (Anlage) zur Aufreinigung für die Rohdinitrotoluole R-DNT, insbesondere eine wie zuvor beschriebene Vorrichtung (Anlage) nach der vorliegenden Erfindung, umfassend:
   (a) mindestens eine Wascheinheit WE zur Wäsche der Rohdinitrotoluole R-DNT mit mindestens einem Waschmedium WM; und
   (b) stromabwärts zur Wascheinheit WE angeordnet, mindestens eine Strippeinrichtung (Strippvorrichtung) SK zur Strippung (Strippgasbehandlung) der aus der Wascheinheit WE hervorgehenden gewaschenen Dinitrotoluole W-DNT; DNT-I, DNT-II, DNT-III mit mindestens einem gegenüber Dinitrotoluolen nichtreaktiven und/oder gegenüber Dinitrotoluolen inerten Gas (Strippgas) SG, ausgewählt ist aus der Gruppe von Stickstoff, Sauerstoff, Edelgasen, Wasserstoff, Kohlenstoffoxiden und deren Mischungen, in Abwesenheit von Wasserdampf.

Bezüglich weitergehender Einzelheiten zu der erfindungsgemäßen Produktionsanlage kann zur Vermeidung unnötiger Wiederholungen auf die obigen Ausführungen zu dem erfindungsgemäßen Verfahren und zu der erfindungsgemäßen Vorrichtung (Anlage) verwiesen werden, welche in Bezug auf die erfindungsgemäße Produktionsanlage entsprechend gelten.

Die nun nachfolgenden Ausführungen beziehen sich sowohl auf die erfindungsgemäße Vorrichtung (Anlage) als auch auf die erfindungsgemäße Produktionsanlage.

Sowohl in Bezug auf die erfindungsgemäße Vorrichtung (Anlage) als auch in Bezug auf die erfindungsgemäße Produktionsanlage kann es vorgesehen sein, dass die Strippeinrichtung SK zusätzlich einen Wärmetauscher WM1 zur Temperierung der gewaschenen Dinitrotoluole W-DNT; DNT-I, DNT-II, DNT-III vor Einbringung in die Strippeinrichtung (SK) umfasst. Zudem kann es vorgesehen sein, dass die Strippeinrichtung SK zusätzlich einen Wärmetauscher WM2 zur Temperierung des Strippgases SG vor Einbringung in die Strippeinrichtung SK umfasst.

Zudem kann es sowohl in Bezug auf die erfindungsgemäße Vorrichtung (Anlage) als auch in Bezug auf die erfindungsgemäße Produktionsanlage vorgesehen sein, dass, stromabwärts zur Strippeinrichtung SK angeordnet, eine Abgasbehandlungseinrichtung für die Aufreinigung und/oder Behandlung des mit aus den Dinitrotoluolen entfernten Verunreinigungen beladenen Strippgases vorgesehen ist. Gemäß einer Ausführungsform kann die Abgasbehandlungseinrichtung eine Wascheinrichtung WK, insbesondere eine Waschkolonne, gegebenenfalls mit nachgeschalteter, vorzugsweise thermischer Abgasbehandlungsanlage AB, umfassen. Gemäß einer alternativen Ausführungsform kann die Abgasbehandlungseinrichtung (nur) eine vorzugsweise thermische Abgasbehandlungsanlage AB umfassen (d. h. ohne vorgeschaltete Wascheinrichtung).

Weitere Vorteile, Eigenschaften Aspekte und Merkmale der vorliegenden Erfindung ergeben sich aus der nachfolgenden Beschreibung der in den Figurendarstellungen gemäß Figs.1 bis 4 dargestellten, erfindungsgemäß bevorzugten Ausführungsformen.

Es zeigt:
- Fig. 1:: eine schematische Darstellung eines erfindungsgemäßen Verfahrens und einer erfindungsgemäßen Vorrichtung (Anlage) zur Behandlung von einem zuvor mit einem Waschmedium behandelten DNT mit einem Strippgas gemäß einer bevorzugten Ausführungsform der vorliegenden Erfindung;

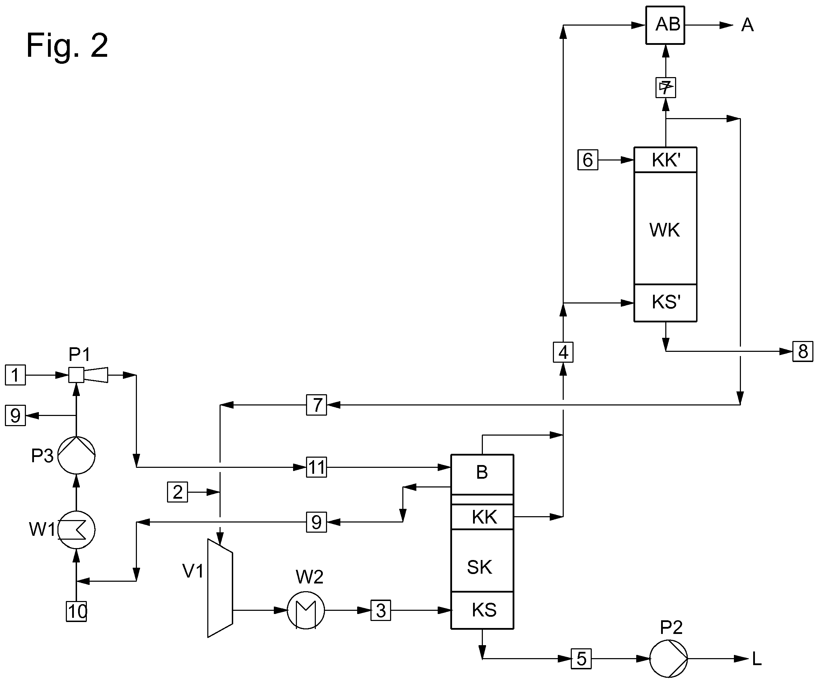

- Fig. 2: eine schematische Darstellung eines erfindungsgemäßen Verfahrens und einer erfindungsgemäßen Vorrichtung (Anlage) zur Strippung von einem zuvor mit einem Waschmedium behandelten DNT, welches mittels eines Injektors der Strippeinheit zugeführt wird, gemäß einer weiteren bevorzugten Ausführungsform der vorliegenden Erfindung;

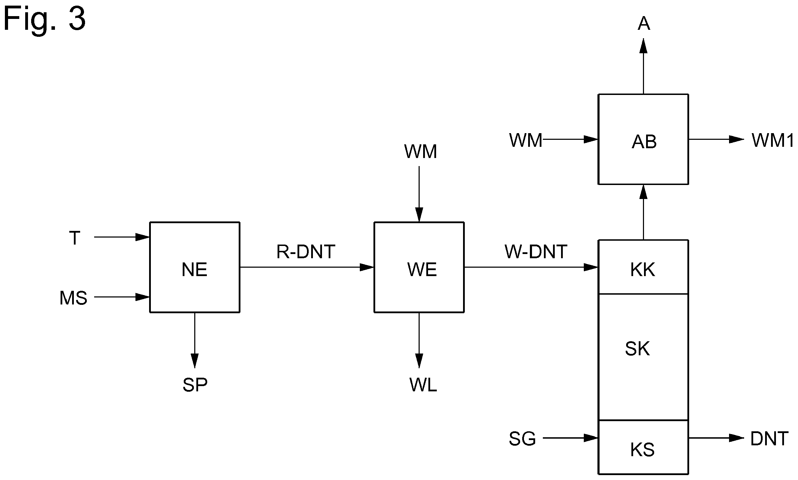

- Fig. 3: eine schematische Darstellung einer erfindungsgemäßen Produktionsanlage mit einer Nitrierung und einer nachfolgenden Aufreinigung mittels Wäsche des DNT in Verbindung mit einer der Wäsche nachgeschalteten Strippvorrichtung gemäß einer bevorzugten Ausführungsform der vorliegenden Erfindung;

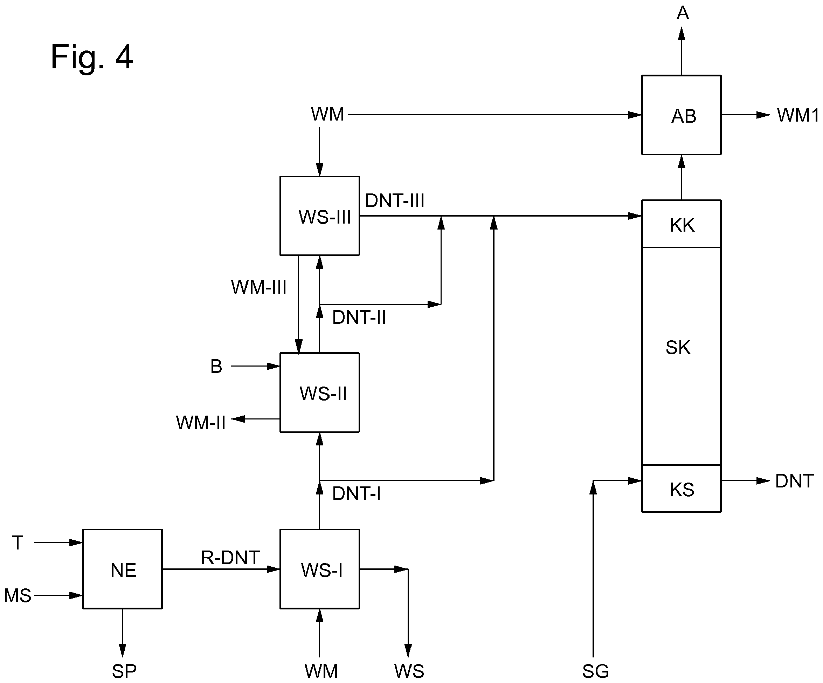

- Fig. 4: eine schematische Darstellung einer erfindungsgemäßen Produktionsanlage mit einer Nitrierung und einer nachfolgenden Aufreinigung mittels Wäsche des DNT mit drei Waschschritten in Verbindung mit einer der Wäsche nachgeschalteten Strippvorrichtung gemäß einer weiteren bevorzugten Ausführungsform der vorliegenden Erfindung.

Fig. 1 zeigt ein allgemeines Beispiel für die Strippung von DNT mit einem Inertgas. Dabei kann das zu strippende Gut (DNT) beispielsweise gravimetrisch oder aber mit einer zum Fördern von DNT geeigneten Fördereinheit der Strippeinheit zugeführt werden. Das zuvor mit einem Waschmedium behandelte DNT 1 kann dabei entweder gravimetrisch oder mit einer zum Fördern von DNT geeigneten Pumpe P1 einem Wärmetauscher W1 zugeführt werden. Nach dem Wärmetauscher gelangt das DNT 1 mit der für die Strippbehandlung vorgesehenen Temperatur auf den Kopf KK einer Strippkolonne SK. Im unteren Teil der Strippkolonne SK, dem Kolonnensumpf KS, wird gleichzeitig das temperierte Strippgas 3 eingespeist, welches mittels eines Wärmetauschers W2 auf die für die Strippbehandlung vorgesehene Temperatur eingestellt wird. Nach gravimetrischem Durchlauf des Strippgutes (DNT 1) durch die Strippkolonne SK wird das von den leichtflüchtigen Verunreinigungen befreite DNT 5 aus dem Kolonnensumpf KS abgezogen und fließt entweder gravimetrisch oder mittels einer zum Fördern von DNT geeigneten Pumpe P2 in das Zwischenlager L für das gereinigte DNT 5.

Das mit den aus dem DNT abgetrennten leichtflüchtigen Verbindungen beladene Strippgas 4 wird im unteren Teil KS' einer Waschkolonne WK eingespeist und mit dem im Kolonnenkopf KK' der Waschkolonne WK aufgegebenen Waschmedium 6 gekühlt (z. B. auf Umgebungstemperatur) und behandelt. Das von den kondensierbaren und auswaschbaren leichtflüchtigen Verunreinigungen (wie DNT, Blausäure, Salpetersäure, leichtflüchtige aromatische oder aliphatische Kohlenwasserstoffe, Lösungsmittelreste etc.) befreite Strippgas 7 wird entweder im Kreis geführt oder direkt oder ein Teilstrom davon an eine Abgasbehandlungsanlage AB, z. B. eine thermische Abgasbehandlung, abgegeben und gelangt von dort als gereinigtes Abgas A in die Umwelt. Bei Rückführung des Strippgases 7 oder eines Teilstromes davon wird der ausgeschleuste Teil durch Frischgas 2 ersetzt und über ein Gebläse V1 in den Kreislauf zurückgeführt. Das aus dem Kolonnensumpf KS' der Waschkolonne WK abgezogene, mit den abgetrennten kondensierbaren und auswaschbaren Verunreinigungen beladene Waschmedium 8 wird - nach Abtrennung des gelösten und suspendierten Produktes, z. B. durch Extraktion mit dem zu nitrierenden Aromaten - zusammen mit den anderen Abwässern aus der Reinigung des Roh-DNT so aufbereitet, dass es abgegeben oder in den Nitrierkreislauf zurückgeführt werden kann.

Fig. 2 zeigt eine spezielle Ausführungsform des in Fig. 1 allgemein beschriebenen Verfahrens bzw. der erfindungsgemäßen Vorrichtung mit einem Injektor zum Transport des DNT: Das mit einem Waschmedium behandelte DNT 1 wird aus der Wascheinrichtung mit einem Injektor P1 als Emulsion zu der Strippkolonne SK transportiert. Als Treibmittel für den Injektor P1 dient das jeweilige Waschmedium aus der vorgeschalteten Wäsche, bevorzugt das Waschmedium 10, welches den zuvor beschriebenen Waschschritten I bis III (saure, basische und neutrale Wäsche) zugeführt wird. Die Emulsion 11 aus DNT/Treibmittel wird in den Phasentrennapparat B transportiert, welcher als Oberteil der Strippkolonne SK ausgebildet sein kann. Das abgetrennte Transportmedium 9, gesättigt mit dem Nitroaromaten (DNT) und enthaltend noch Spuren an im DNT gelösten Verunreinigungen, wird nach Zumischung der vorgesehenen Menge Waschmedium 10 und nach Durchlauf durch den Wärmetauscher W1 als Treibmedium für den Injektor mittels Pumpe P1 in den Transportkreislauf zurückgeführt. Das überschüssige Wasser 9 aus dem Treibmittelkreislauf wird als Waschmedium in die Wäsche eingespeist.

Das im Phasentrennapparat B abgetrennte DNT fließt gravimetrisch auf den Kopf KK der Strippkolonne SK. Im unteren Teil der Strippkolonne SK, dem Kolonnensumpf KS, wird gleichzeitig das temperierte Strippgas 3 eingespeist, welches durch den Wärmetauscher W2 auf für die Strippbehandlung vorgesehene Temperatur eingestellt wird. Nach gravimetrischem Durchlauf des Strippgutes (DNT) durch die Strippkolonne SK wird das von den leichtflüchtigen Verunreinigungen befreite DNT 5 aus dem Kolonnensumpf abgezogen und fließt entweder gravimetrisch oder mittels einer zum Fördern von DNT geeigneten Pumpe P2 in das Zwischenlager L für das gereinigte DNT 5.

Die Behandlung des Strippgases nach der Strippkolonne SK, beladen mit den abgetrennten flüchtigen Verunreinigungen, erfolgt analog wie für Fig.1 beschrieben.

Fig. 3 zeigt eine allgemeine Ausführung einer erfindungsgemäßen Produktionsanlage zur Herstellung von DNT aus einer adiabatischen oder isothermen Nitrierung von Toluol mit integrierter Behandlung des gewaschenen DNT mit einem Strippgas.

Das in der Nitriereinheit NE durch Umsetzung von Toluol T mit einem Salpetersäure/Schwefelsäure-Nitriersäuregemisch MS gebildete Roh-DNT R-DNT wird nach Abtrennung der Nitrierendsäure SP in der Wascheinheit WE (z. B. zur Durchführung der zuvor beschriebenen Waschschritte I bis III) mit einem Waschmedium WM, bevorzugt einer Waschsäure, einer Waschlauge oder Wasser, gewaschen.

Die resultierenden Abwässer WL aus der Wascheinheit WE werden entsprechend ihrer Herkunft, wie vorstehend beschrieben, aufgearbeitet.

Das aus der Wascheinheit WE abgetrennte DNT W-DNT, aus welchem bevorzugt die starken Mineralsäuren, wie Schwefelsäure und Salpetersäure (Waschschritt I) und alle mittelstarken Säuren mit einem pKₛ-Wert bis 8, wie Trinitrokresole, Nitrobenzoesäuren, Oxalsäure, CO₂ etc., extrahiert worden sind, aber welches noch die schwachen Säuren, wie Blausäure, und die Inertstoffe, wie NO, CO, N₂O oder aliphatische oder aromatische Kohlenwasserstoffe, enthält, wird als Schmelze auf den Kopf KK der erfindungsgemäß eingesetzten Strippeinrichtung/Strippkolonne SK aufgegeben und mit dem Strippgas SG, welches im Unterteil/Kolonnensumpf KS der Strippeinheit SK eingeleitet wird, behandelt. Das DNT läuft z. B. gravimetrisch durch die Strippeinheit SK und wird am Unterteil KS der Strippeinheit SK als reines Produkt DNT entnommen. Das Strippgas, beladen mit den aus dem DNT entfernten Verunreinigungen, wird in einer Abgasbehandlungsanlage AB, z. B. durch Wäsche mit einem Waschmedium und/oder weiteren Abgasbehandlungen, wie einer Entfernung von NOx, und/oder einer thermischen Nachbehandlung derart aufgereinigt, dass ein den jeweils gültigen Umweltvorschriften entsprechendes Abgas A abgegeben werden kann. Das Waschmedium WM1, welches am Boden KS der Waschkolonne WK abgezogen wird, enthält - neben Spuren von DNT - in gelöster oder suspendierter Form alle sonstigen kondensierbaren Verunreinigungen, welche aus dem DNT in der Strippeinrichtung SK entfernt worden sind. Vor Abgabe des Waschmediums WM1 in einen Vorfluter werden diese Stoffe noch aus dem Waschmedium WM1 entfernt.

Fig. 4 zeigt eine spezielle Ausführungsform einer erfindungsgemäßen Produktionsanlage zur Herstellung von DNT aus einer adiabatischen oder isothermen Nitrierung von Toluol gemäß Fig. 3 mit den üblichen drei Waschschritten einer Wäsche des DNT in Verbindung mit der integrierten, erfindungsgemäß vorgesehenen Behandlung des in den Waschschritten I bis III gewaschenen DNT mit einem Strippgas. Das in der Nitriereinheit NE durch Umsetzung von Toluol T mit Salpetersäure in Gegenwart von Schwefelsäure MS gebildete Roh-DNT R-DNT wird nach Abtrennung der Nitrierendsäure SP in drei Waschschritten WS-I, WS-II und WS-III, wie zuvor beschrieben, gewaschen.

In Waschschritt I WS-I (saure Wäsche) werden mit dem zugeführten Waschmedium WM vor allem Schwefelsäure und Salpetersäure aus dem Roh-DNT abgetrennt, wobei das Verhältnis von Waschmedium WM zu zu waschendem Produkt R-DNT derart gewählt wird, dass die Waschsäure WS einen Gehalt an Säuren von 10 % bis 40 Gew.-% enthält. Nach Phasentrennung wird die Waschsäure WS direkt oder nach Aufkonzentrierung in die Nitrierung zurückgeführt. Das aus dem Waschschritt I WS-I ausgeschleuste DNT DNT-I wird in den Waschschritt II WS-II (alkalische Wäsche) eingespeist oder aber mit einem Strippgas SG behandelt.

In Waschschritt II WS-II werden durch Zusatz einer Base B z. B. zu dem aus Waschschritt III WS-III zugeführten Waschmedium WM-III bei einem pH-Wert des Wachmediums im Bereich von 8,5 bis 9,5 die mittelstarken und schwachen Säuren, wie Trinitrokresole, Nitrobenzoesäuren, CO₂, Oxalsäure etc., abgetrennt. Nach Phasentrennung wird das alkalische Waschmedium WM-II in eine Abwasserbehandlung abgegeben. Das aus dem Waschschritt II WS-II ausgeschleuste DNT DNT-II wird in den Waschschritt III WS-III (Neutralwäsche) eingespeist oder aber mit einem Strippgas SG behandelt.

In Waschschritt III WS-III (Neutralwäsche) werden mit dem zugeführten Waschmedium WM Spuren von Waschmedium WM-II und noch vorliegende Spuren an schwer extrahierbaren Verunreinigungen abgetrennt. Nach Phasentrennung wird das Waschmedium WM-III in den Waschschritt WS-II als Waschmedium eingespeist. Das aus dem Waschschritt III WS-III ausgeschleuste DNT DNT-III wird mit einem Strippgas SG behandelt.

Das aus den drei Waschschritten WS-I, WS-II und WS-III abgetrennten DNTs DNT-I, DNT-II und DNT-III werden als Schmelze auf den Kopf KK der erfindungsgemäß eingesetzten Strippeinrichtung (Strippkolonne) SK aufgegeben und mit dem Strippgas SG, welches im Unterteil (Kolonnensumpf) KS der Strippeinheit SK eingeleitet wird, behandelt. Das DNT (DNT-I bis DNT-III) läuft z. B. gravimetrisch durch die Strippeinheit SK und wird am Unterteil KS der Strippeinheit SK als reines Produkt (DNT) entnommen. Das Strippgas, beladen mit den aus dem DNT entfernten Verunreinigungen, wird in einer Abgasbehandlungsanlage AB durch Wäsche mit einem Waschmedium und/oder weiteren Abgabehandlungen, wie einer Entfernung von NOx, und/oder einer thermischen Nachbehandlung derart gereinigt, dass ein den jeweils gültigen Umweltvorschriften entsprechendes Abgas A abgegeben werden kann.

Das Waschmedium WM1, das am Boden KS der Waschkolonne abgezogen wird, enthält neben Spuren von DNT auch noch in gelöster oder suspendierter Form alle sonstigen kondensierbaren Verunreinigungen, welche aus dem DNT in der Strippeinrichtung SK entfernt worden sind. Vor Abgabe des Waschmediums WM1 in einen Vorfluter müssen diese Stoffe noch entfernt werden.

Weitere Ausgestaltungen, Abwandlungen, Variationen, Modifikationen oder dergleichen der vorliegenden Erfindung sind für den Fachmann beim Lesen der Beschreibung ohne Weiteres erkennbar und realisierbar, ohne dass der Rahmen der vorliegenden Erfindung verlassen wird.

Die vorliegende Erfindung wird anhand der folgenden Ausführungsbeispiele veranschaulicht, ohne jedoch die vorliegende Erfindung hierauf zu beschränken.

### AUSFÜHRUNGSBEISPIELE:

### Beispiel 1 (Vergleich)

Ein Roh-DNT (2.900 kg/Stunde) mit ca. 14 kg Salpetersäure, ca. 7,4 kg an Stickstoffdioxid, ca. 0,37 kg Schwefelsäure und ca. 50 ppm an Blausäure pro Tonne Roh-DNT wurde entsprechend dem Stand der Technik in drei Waschschritten gewaschen.

Im ersten Waschschritt (saure Wäsche) wurde entsprechend der EP 0 736 514 A1 bzw. der EP 1 780 198 A1 mit einem DNT/Wasser-Verhältnis von ca. 9:1 (Gewichtsbasis) gewaschen. Zur Aufrechterhaltung des vorgegebenen DNT/Waschsäure-Phasenverhältnisses in den Waschapparaten wurde die Waschsäure zusätzlich im Kreis geführt. Die Waschsäure aus dieser sauren Wäsche mit ca. 27,6 % Gesamtsäure wurde nach Aufkonzentrierung auf ca. 60 % Gesamtsäure (als Salpetersäure bestimmt) in die Nitrierung zurückgeführt. Das Brüdenkonzentrat aus der Aufkonzentrierung wurde in die saure Wäsche zurückgeführt.

Im zweiten Waschschritt (alkalische Wäsche) wurden in Gegenwart von Soda bei pH 9,2, alle Nitrokresole, Nitrobenzoesäuren und alle schwache Säuren mit pKₛ-Werten um zwei Einheiten kleiner als der pH-Wert der Waschlauge vollständig aus dem DNT extrahiert. Neutralstoffe, wie CO, N₂O, Kohlenwasserstoffe etc., und sehr schwache Säuren, wie Blausäure mit einem pKₛ-Wert von 9,2, wurden nur entsprechend ihrer Löslichkeit bzw. nur teilweise in das Waschmedium extrahiert. Zur Aufrechterhaltung des vorgegebenen DNT/Waschmedium-Phasenverhältnisses in den Waschapparaten wurde die Waschlauge zusätzlich im Kreis geführt

Im dritten Waschschritt (Neutralwäsche) wurde mit einem DNT/Prozesswasser-Verhältnis von 2 : 1 (Gewichtsbasis) gewaschen. Zur Aufrechterhaltung des vorgegebenen DNT/Waschmedium-Phasenverhältnisses in den Waschapparaten wurde das Waschwasser zusätzlich im Kreis geführt. Das Waschwasser aus der Neutralwäsche wurde nach Phasentrennung in die alkalische Wäsche eingespeist.

Nach dieser Wäsche gemäß dem Stand der Technik wurde ein DNT erhalten, aus welchem alle im alkalischen Bereich Salze bildenden Säuren weitgehend abgetrennt wurden. Neben max. 2 ppm Sulfat, weniger als 10 ppm Nitrokresolen (vorwiegend Trinitrokresolen) und weniger als 10 ppm an Nitrobenzoesäuren wurden noch ca. 15 ppm Salpetersäure (Summe aus HNO₃ plus HNO₂), ca. 2 ppm Blausäure, ca. 100 ppm CO, ca. 5 ppm CO₂, 50 ppm N₂O, ca. 300 ppm aliphatische Kohlenwasserstoffe und ca. 1 % Wasser gefunden.

### Beispiel 2 (erfindungsgemäß)

Ein entsprechend dem Stand der Technik gewaschenes DNT (2.800 kg/h), wie in Beispiel 1 beschrieben, wurde mit 85 °C auf den Kopf einer Strippkolonne mit 10 Böden aufgegeben und mit 45 Normkubikmeter/h Stickstoff mit einer Temperatur von ebenfalls 85 °C gestrippt. Das am Boden der Kolonne ablaufende DNT (Temperatur: ca. 74 °C) enthielt noch weniger als 2 ppm Salpetersäure (Summe HNO₃ plus HNO₂), weniger als 0,1 ppm an Blausäure und weniger als 5 ppm an N₂O, CO und CO₂ in Summe (neben noch weniger als 10 ppm an aliphatischen Kohlenwasserstoffen). Der Wassergehalt sank auf ca. 0,17 %. Im Abgas befand sich neben den ausgestrippten Verunreinigungen noch DNT, das bei einer Zwischenkondensation bzw. einer Behandlung mit einem Waschmedium abgeschieden wurde.

Wie das erfindungsgemäße Ausführungsbeispiel 2 belegt, kann die Reinheit der erhaltenen DNT gegenüber dem Aufreinigungsverfahren gemäß dem Stand der Technik (Beispiel 1) signifikant verbessert werden; insbesondere können erfindungsgemäß auch die nach dem Stand der Technik nicht entfernbaren Verunreinigungen auf einfache wie effiziente Weise entfernt werden.

### Beispiel 3 (erfindungsgemäß)

Ein Roh-DNT (2.900 kg/Stunde) mit ca. 14 kg Salpetersäure, ca. 7,4 kg an Stickstoffdioxid, ca. 0,37 kg Schwefelsäure und ca. 50 ppm an Blausäure pro Tonne Roh-DNT wurde in nur einem Waschschritt (nur saure Wäsche) entsprechend der EP 0 736 514 A1 bzw. der EP 1 780 198 A1 mit einem DNT/Prozesswasser-Verhältnis von ca. 9:1 (Gewichtsbasis) gewaschen. Zur Aufrechterhaltung des vorgegebenen DNT/Waschsäure-Phasenverhältnisses in den Waschapparaten wurde die Waschsäure zusätzlich im Kreis geführt. Die Waschsäure aus dieser sauren Wäsche mit ca. 27,6 % Gesamtsäure und ca. 80 ppm an Blausäure wurde nach Aufkonzentrierung auf ca. 60 % Gesamtsäure (als Salpetersäure bestimmt) in die Nitrierung zurückgeführt. Das Brüdenkondensat aus der Aufkonzentrierung mit ca.1,5 % Gesamtsäure (als Salpetersäure bestimmt) und ca. 100 ppm an Blausäure wurde durch Extraktion mit Toluol von noch gelöstem DNT befreit; das Extraktionsmittel wurde anschließend zusammen mit anderen flüchtigen Stoffen, wie z. B. Blausäure, durch Strippen mit Dampf aus dem Brüdenkonzentrat entfernt, und das auf diese Weise behandelte Brüdenkondensat wurde nach Neutralisation direkt in eine biologische Nachbehandlung abgegeben.

Das DNT nach dieser sauren Wäsche (ca. 2.800 kg/h) enthielt noch alle im Roh-DNT vorliegenden Nitrokresole (vorwiegend Trinitrokresole) und Nitrobenzoesäuren, ca. 2 ppm Sulfat, 650 ppm Salpetersäure (Summe aus HNO₃ plus HNO₂), 40 ppm Blausäure, 150 ppm CO, 40 ppm CO₂, 60 ppm N₂O, ca. 300 ppm aliphatische Kohlenwasserstoffe und ca. 1 % Wasser.

Dieses DNT wurde mit 85 °C auf den Kopf einer Strippkolonne mit 10 Böden aufgegeben und mit 80 Normkubikmeter/h Stickstoff mit einer Temperatur von ebenfalls 85 °C gestrippt. Das am Boden der Kolonne ablaufende DNT (Temperatur ca. 71 °C) enthielt noch ca. 30 ppm an Salpetersäure (Summe HNO₃ plus HNO₂), max. 0,5 ppm Blausäure und weniger als 10 ppm an N₂O, CO und CO₂ in Summe und weniger als 10 ppm an aliphatischen Kohlenwasserstoffen. Der Wassergehalt sank auf ca. 0,2 %. Im Abgas befand sich neben den ausgestrippten Verunreinigungen noch DNT, welches bei einer Zwischenkondensation abgeschieden und zurückgeführt wurde.

Wie das erfindungsgemäße Ausführungsbeispiel 3 belegt, kann durch die erfindungsgemäße Vorgehensweise selbst bei nur einstufiger Wäsche die Reinheit der erhaltenen DNT gegenüber herkömmlichen Aufreinigungsverfahren des Standes der Technik signifikant verbessert werden.

### Beispiel 4 (Vergleich)

Beispiel 3 wurde wiederholt, jedoch mit der Abweichung bzw. Umkehr der Reihenfolge der Behandlungsschritte (vorgeschaltete Strippgasbehandlung mit nachfolgender Wäsche):
Zunächst wurde die Strippgasbehandlung entsprechend Beispiel 3 durchgeführt und erst anschließend die saure Wäsche entsprechend Beispiel 3. Es fielen zwei Salpetersäureabfallströme an (nämlich im Abgas der Strippung und im Waschwasser). Das so erhaltene DNT enthielt - neben allen auch im Roh-DNT vorliegenden Nitrokresolen (vorwiegend Trinitrokresole) und Nitrobenzoesäuren - noch ca. 3,0 ppm Sulfat, 1.200 ppm Salpetersäure (Summe aus HNO₃ plus HNO₂), 35 ppm Blausäure, 110 ppm CO, 30 ppm CO₂, 35 ppm N₂O, ca. 250 ppm aliphatische Kohlenwasserstoffe und ca. 1,2 % Wasser. Wie das Beispiel 4 belegt, kann bei abweichender Behandlungsreihenfolge (Strippung vor Wäsche) keine effiziente Reinigungswirkung erzielt werden.

## Patentansprüche

1. Verfahren zur Aufreinigung von aus der Nitrierung von Toluol in Gegenwart eines Salpetersäure/Schwefelsäure-Nitriersäuregemisches resultierenden Rohdinitrotoluolen, insbesondere zur Entfernung auch von insbesondere flüchtigen Verunreinigungen aus den Rohdinitrotoluolen,
**dadurch gekennzeichnet,**
(a) **dass** die Rohdinitrotoluole nach Abtrennung des Nitrierendsäuregemisches zunächst einer Wäsche mit mindestens einem Waschmedium unterzogen werden, gefolgt von einer Abtrennung des Waschmediums; und
(b) **dass** nachfolgend die aus Verfahrensschritt (a) resultierenden gewaschenen Dinitrotoluole einer Strippung (Strippgasbehandlung) mit mindestens einem Gas (Strippgas) unterzogen werden, wobei als Strippgas ein gegenüber Dinitrotoluolen nichtreaktives und/oder gegenüber Dinitrotoluolen inertes Gas eingesetzt wird, wobei das Strippgas ausgewählt ist aus der Gruppe von Stickstoff, Sauerstoff, Edelgasen, Wasserstoff, Kohlenstoffoxiden und deren Mischungen, und wobei die Strippung in Abwesenheit von Wasserdampf durchgeführt wird.

2. Verfahren nach Anspruch 1,
wobei in Verfahrensschritt (a) die Wäsche als flüssig/flüssig-Extraktion durchgeführt wird; und/oder
wobei in Verfahrensschritt (a) die Wäsche in ein oder mehreren Waschschritten, insbesondere in ein, zwei oder drei Waschschritten, durchgeführt wird, insbesondere wobei jeder Waschschritt ein oder mehrere Wasch- und/oder Extraktionsstufen umfassen kann und/oder insbesondere wobei jeder Waschschritt einstufig oder mehrstufig, insbesondere einstufig, zweistufig oder dreistufig, durchgeführt werden kann; und/oder
wobei in Verfahrensschritt (a) die Wäsche in drei Waschschritten durchgeführt wird, wobei die Wäsche in drei Waschschritten einen ersten sauren Waschschritt ("saurer Waschschritt"), einen nachfolgenden zweiten basischen (alkalischen) Waschschritt ("basischer oder alkalischer Waschschritt") und einen abschließenden dritten neutralen Waschschritt ("Neutralwaschschritt") umfasst; und/oder
wobei in Verfahrensschritt (a) die Wäsche, insbesondere in jedem Waschschritt, im Querstrom oder im Gegenstrom oder in einer Kombination von Quer- und Gegenstrom durchgeführt wird, vorzugsweise unter Rezyklierung des Waschmediums; und/oder wobei in Verfahrensschritt (a) die Abtrennung des Waschmediums mittels einer Scheidevorrichtung (Scheider), insbesondere einer statischen Scheidevorrichtung, oder mittels eines Zentrifugalseparators erfolgt.

3. Verfahren nach einem der vorangehenden Ansprüche,
wobei in Verfahrensschritt (b) die Strippung in einer Strippungsvorrichtung durchgeführt wird, insbesondere wobei als Strippungsvorrichtung eine Strippkolonne, insbesondere eine ein- oder mehrstufige Strippkolonne, bevorzugt eine Füllkörper-, Siebboden-, Glockenboden- oder Fallfilmkolonne oder Kombinationen hiervon, oder aber ein Dünnschichtverdampfer, ein Gas/Flüssig-Reaktor oder eine Horizontalstrippvorrichtung eingesetzt wird; und/oder
wobei in Verfahrensschritt (b) die Strippung im Quer- und/oder Gegenstrom, bevorzugt im Gegenstrom, erfolgt und/oder wobei in Verfahrensschritt (b) das Strippgas im Quer- und/oder Gegenstrom, bevorzugt im Gegenstrom, geführt wird; und/oder
wobei in Verfahrensschritt (b) die Strippung mehrstufig erfolgt, insbesondere wobei in den verschiedenen Strippungsstufen die Strippung jeweils im Quer- oder Gegenstrom, bevorzugt im Gegenstrom, erfolgt und/oder insbesondere wobei in den verschiedenen Strippungsstufen das Strippgas jeweils im Quer- oder Gegenstrom, bevorzugt im Gegenstrom, geführt wird.

4. Verfahren nach einem der vorangehenden Ansprüche,
wobei in Verfahrensschritt (b) die Strippung bei Temperaturen oberhalb der Schmelztemperatur der Dinitrotoluole, insbesondere bei einer Temperatur von mindestens 56 °C, vorzugsweise bei einer Temperatur von mindestens 60 °C, bevorzugt bei einer Temperatur von mindestens 65 °C, durchgeführt wird und/oder wobei in Verfahrensschritt (b) die Strippung in einem Temperaturbereich von 56 °C bis 130 °C, insbesondere in einem Temperaturbereich von 65 °C bis 105 °C, vorzugsweise in einem Temperaturbereich von 70 °C bis 95 °C, durchgeführt wird; und/oder
wobei in Verfahrensschritt (b) das Strippgas mit einer Strippgastemperatur im Bereich von 65 °C bis 140 °C, insbesondere im Bereich von 70 °C bis 120 °C, vorzugsweise im Bereich von 70 °C bis 95 °C, eingesetzt wird und/oder mit den Dinitrotoluolen in Kontakt gebracht wird und/oder wobei in Verfahrensschritt (b) das Strippgas bei Einspeisung in die Strippvorrichtung eine Temperatur im Bereich von 65 °C bis 140 °C, insbesondere im Bereich von 70 °C bis 120 °C, vorzugsweise im Bereich von 70 °C bis 95 °C, aufweist; und/oder
wobei in Verfahrensschritt (b), vorzugsweise während des gesamten Verfahrens, die Dinitrotoluole im flüssigen Aggregatzustand und/oder oberhalb ihrer Schmelztemperatur vorliegen und/oder wobei in Verfahrensschritt (b), vorzugsweise während des gesamten Verfahrens, die Dinitrotoluole eine Temperatur von mindestens 60 °C aufweisen und bevorzugt eine Temperatur im Bereich von 56 °C bis 130 °C, insbesondere im Bereich von 60 °C bis 105 °C, vorzugsweise im Bereich von 65 °C bis 95 °C, besonders bevorzugt im Bereich von 65 °C bis 85 °C, aufweisen; und/oder
wobei in Verfahrensschritt (b) die Strippung bei Atmosphärendruck (Normaldruck; 1,01325 bar), im Vakuum (Unterdruck) oder bei Überdruck, vorzugsweise bei Atmosphärendruck, durchgeführt wird und/oder wobei in Verfahrensschritt (b) die Strippung bei einem Absolutdruck im Bereich von 0,2 bar bis 3 bar, insbesondere im Bereich von 0,3 bar bis 1,2 bar, vorzugsweise im Bereich von 0,4 bar bis 1,1 bar, besonders bevorzugt bei etwa 1 bar, durchgeführt wird.

5. Verfahren nach einem der vorangehenden Ansprüche,
wobei in Verfahrensschritt (b) die eingesetzte Menge an Strippgas in Abhängigkeit von den Bedingungen der Strippung, insbesondere Temperatur und/oder Druck und/oder Dauer der Strippung und/oder Art des Strippgases, und/oder in Abhängigkeit von dem Gehalt und/oder Art an Verunreinigungen in den in Verfahrensschritt (b) zu behandelnden Dinitrotoluolen und/oder in Abhängigkeit von dem angestrebten Restgehalt an Verunreinigungen in den nach Verfahrensschritt (b) resultierenden Dinitrotoluolen gewählt und/oder eingestellt wird; und/oder
wobei in Verfahrensschritt (b) die eingesetzte Menge an Strippgas, bezogen auf 1 Tonne zu behandelnder Dinitrotoluole und berechnet als Volumen an Strippgas in Normkubikmetern unter Standardbedingungen (Druck von 1,01325 bar; Luftfeuchtigkeit von 0 %, d. h. trockenes Strippgas; Temperatur von 0 °C [Normbedingungen nach DIN 1343, STPD]), 0,1 bis 1.000 Normkubikmeter Strippgas, insbesondere 0,5 bis 500 Normkubikmeter, vorzugsweise 1 bis 200 Normkubikmeter, besonders bevorzugt 2 bis 120 Normkubikmeter, ganz besonders bevorzugt 5 bis 80 Normkubikmeter, noch mehr bevorzugt 10 bis 60 Normkubikmeter, beträgt; und/oder
wobei in Verfahrensschritt (b) die eingesetzte Menge an Strippgas, bezogen auf 1 Tonne zu behandelnder Dinitrotoluole und berechnet als Menge an Stickstoff als Strippgas unter Standardbedingungen (Druck von 1,01325 bar; Luftfeuchtigkeit von 0 %, d. h. trockenes Strippgas; Temperatur von 0 °C [Normbedingungen nach DIN 1343, STPD]), 0,5 bis 500 kg, insbesondere 1 bis 250 kg, vorzugsweise 1 bis 140 kg, besonders bevorzugt 5 bis 50 kg, noch mehr bevorzugt 10 bis 40 kg, beträgt; und/oder
wobei in Verfahrensschritt (b) (gleichermaßen) eine Reduktion des Wassergehalts der behandelten Dinitrotoluole erfolgt, insbesondere wobei der Wassergehalt in den nach Verfahrensschritt (b) resultierenden Dinitrotoluolen höchstens 1 Gew.-%, insbesondere höchstens 0,8 Gew.-%, vorzugsweise höchstens 0,5 Gew.-%, besonders bevorzugt höchstens 0,4 Gew.-%, ganz besonders bevorzugt höchstens 0,3 Gew.-%, noch mehr bevorzugt höchstens 0,2 Gew.-%, jeweils bezogen auf die Dinitrotoluole, beträgt;
und/oder
wobei in Verfahrensschritt (b) der Gehalt an Wasser in den resultierenden Dinitrotoluolen derart abgesenkt wird, dass bei Abkühlung der flüssigen Dinitrotoluole bis zu deren Erstarrungspunkt kein Wasser mehr abgeschieden wird; und/oder
wobei in Verfahrensschritt (b) die Strippung nach einer vorangehenden sauren, basischen oder neutralen Wäsche gemäß Verfahrensschritt (a) erfolgt; und/oder wobei nach Durchführung von Verfahrensschritt (b) das mit den aus den Dinitrotoluolen entfernten Verunreinigungen beladene Strippgas einer Abgasbehandlung, insbesondere einer thermischen Abgasbehandlung, unterworfen wird und/oder wobei nach Durchführung von Verfahrensschritt (b) das mit den aus den Dinitrotoluolen entfernten Verunreinigungen beladene Strippgas einer Behandlung zur Entfernung der Verunreinigungen aus dem beladenen Strippgas, vorzugsweise mittels Kondensation und/oder Wäsche mit einem vorzugsweise wässrigen Waschmedium, unterworden wird, gegebenenfalls gefolgt von einer Rezyklierung des aufgereinigten Strippgases in Verfahrensschritt (b).

6. Verfahren nach einem der vorangehenden Ansprüche,
wobei die mittels des Verfahrens aus den Rohdinitrotoluolen zu entfernenden Verunreinigungen ausgewählt sind aus der Gruppe von Mineralsäuren, insbesondere Schwefelsäure, Salpetersäure und Salpetrige Säure, nichtumgesetzten und/oder teilumgesetzen Edukten, Stickstoffoxiden, Kohlenstoffoxiden und nitrierten und nichtnitrierten Reaktionsnebenprodukten sowie Abbau- und Oxidationsprodukten der aus der Nitrierung stammenden Produkte und Nebenprodukte; und/oder wobei die mittels des Verfahrens aus den Rohdinitrotoluolen zu entfernenden Verunreinigungen ausgewählt sind aus der Gruppe von (i) Mineralsäuren, insbesondere Schwefelsäure, Salpetersäure und Salpetrige Säure; (ii) organischen Säuren, insbesondere Ameisensäure, Essigsäure, Oxalsäure, Nitrobenzoesäuren, wie Mono- und Dinitrobenzoesäuren, Pikrinsäure, Blausäure oder dergleichen; (iii) Nitroaromaten, insbesondere Nitrophenolen, wie Mono-, Di- und Trinitrophenolen, und Nitrokresolen, wie Mono-, Di- und Trinitrokresolen; (iv) Stickstoffoxiden (Stickoxiden), insbesondere Stickstoffmonoxid (NO), Stickstoffdioxid (NO₂) und Distickstoffoxid (N₂O); (v) Kohlenstoffoxiden, insbesondere Kohlenmonoxid (CO) und Kohlendioxid (CO₂); (vi) Kohlenwasserstoffen, insbesondere aliphatischen und cycloaliphatischen Kohlenwasserstoffen; (vii) nichtumgesetzten oder teilumgesetzten Edukten, insbesondere Toluol; und/oder
wobei die aus den Rohdinitrotoluolen zu entfernenden flüchtigen Verunreinigungen eine höhere Flüchtigkeit, insbesondere einen höheren Dampfdruck und/oder einen geringeren Siedepunkt, als die aufzureinigenden Rohdinitrotoluole aufweisen, insbesondere bezogen auf den Dampfdruck der Dinitrotoluole unter Standardbedingungen (Druck von 1,01325 bar; Luftfeuchtigkeit von 0% [Normbedingungen nach DIN 1343, STPD]) und/oder insbesondere bezogen auf den Siedepunkt der Dinitrotoluole bei Standardbedingungen (Druck von 1,01325 bar; Luftfeuchtigkeit von 0 % [Normbedingungen nach DIN 1343, STPD]).

7. Vorrichtung (Anlage) zur Aufreinigung von aus der Nitrierung von Toluol in Gegenwart eines Salpetersäure/Schwefelsäure-Nitriersäuregemisches resultierenden Rohdinitrotoluolen nach Abtrennung des Nitrierendsäuregemisches, insbesondere zur Entfernung auch von insbesondere flüchtigen Verunreinigungen aus den Rohdinitrotoluolen, insbesondere Vorrichtung (Anlage) zur Durchführung eines Verfahrens gemäß einem der vorangehenden Ansprüche,
wobei die Vorrichtung umfasst:
(a) mindestens eine Wascheinheit (WE) zur Wäsche der Rohdinitrotoluole (R-DNT) mit mindestens einem Waschmedium (WM); und,
(b) stromabwärts zur Wascheinheit (WE) angeordnet, mindestens eine Strippeinrichtung (Strippvorrichtung) (SK) zur Strippung (Strippgasbehandlung) der aus der Wascheinheit (WE) hervorgehenden gewaschenen Dinitrotoluole (W-DNT; DNT-I, DNT-II, DNT-III) mit mindestens einem gegenüber Dinitrotoluolen nichtreaktiven und/oder gegenüber Dinitrotoluolen inerten Gas (Strippgas) (SG), ausgewählt ist aus der Gruppe von Stickstoff, Sauerstoff, Edelgasen, Wasserstoff, Kohlenstoffoxiden und deren Mischungen, in Abwesenheit von Wasserdampf.

8. Vorrichtung nach Anspruch 7,
wobei die Wascheinheit (WE) ein oder mehrere, insbesondere ein, zwei oder drei, vorzugsweise drei, Wascheinrichtungen (WS-I, WS-II, WS-III) zur Wäsche der Rohdinitrotoluole (R-DNT), insbesondere eine Wascheinrichtung (WS-I) zur Durchführung einer sauren Wäsche und/oder eine Wascheinrichtung (WS-II) zur Durchführung einer alkalischen Wäsche und/oder eine Wascheinrichtung (WS-III) zur Durchführung einer neutralen Wäsche, aufweist; und/oder
wobei die Wascheinheit (WE) drei Wascheinrichtungen (WS-I, WS-II, WS-III) zur Wäsche der Rohdinitrotoluole (R-DNT) aufweist, umfassend eine Wascheinrichtung (WS-I) zur Durchführung einer sauren Wäsche, eine zu der Wascheinrichtung (WS-I) zur Durchführung der sauren Wäsche stromabwärts angeordnete Wascheinrichtung (WS-II) zur Durchführung einer alkalischen Wäsche und eine stromabwärts zu der Wascheinrichtung (WS-II) zur Durchführung der alkalischen Wäsche angeordnete Wascheinrichtung (WS-III) zur Durchführung einer neutralen Wäsche; und/oder
wobei die Wascheinheit (WE) zusätzlich mindestens eine Abtrenneinrichtung zur Abtrennung des gebrauchten und/oder mit den aus den Dinitrotoluolen entfernten Verunreinigungen beladenen Waschmediums (WL) von den gewaschenen Dinitrotoluolen (W-DNT; DNT-I, DNT-II, DNT-III) umfasst.

9. Vorrichtung nach Anspruch 7 oder 8,
wobei die Strippeinrichtung (SK), insbesondere eine Strippkolonne, ein unteres Ende (Unterteil) (KS), insbesondere einen Kolonnensumpf, und ein oberes Ende (Oberteil) (KK), insbesondere einen Kolonnenkopf, aufweist und eine vorzugsweise am unteren Ende (KS) angeordnete Zuführeinrichtung für das Strippgas (SG) und eine vorzugsweise am oberen Ende (KK) angeordnete Zuführeinrichtung für die gewaschenen Dinitrotoluole (W-DNT; DNT-I, DNT-II, DNT-III) umfasst; und/oder
wobei die Strippeinrichtung (SK) als Strippkolonne mit einem Kolonnensumpf (KS) als unterem Ende (Unterteil) und einem Kolonnenkopf (KK) als oberem Ende (Oberteil) ausgebildet ist, insbesondere wobei die Strippkolonne eine vorzugsweise am Kolonnensumpf (KS) angeordnete Zuführeinrichtung für das Strippgas (SG) und eine vorzugsweise am Kolonnenkopf (KK) angeordnete Zuführeinrichtung für die gewaschenen Dinitrotoluole (W-DNT; DNT-I, DNT-II, DNT-III) umfasst; und/oder
wobei die Strippeinrichtung (SK) derart ausgebildet ist, dass die Strippeinrichtung (SK) im Betriebszustand einen Gegen- und/oder Querstromfluss, vorzugsweise einen Gegenstromfluss, von gewaschenen Dinitrotoluolen (W-DNT; DNT-I, DNT-II, DNT-III) einerseits und Strippgas (SG) andererseits ermöglicht.

10. Produktionsanlage zur Herstellung von Dinitrotoluolen, insbesondere Produktionsanlage zur Nitrierung (Dinitrierung) von Toluol zu Dinitrotoluolen in Gegenwart eines Salpetersäure/Schwefelsäure-Nitriersäuregemisches mit nachfolgender Aufreinigung der aus der Nitrierung hervorgehenden Rohdinitrotoluole,
wobei die Produktionsanlage umfasst:
(i) eine Nitriereinrichtung (NE) zur Nitrierung (Dinitrierung) von Toluol (T) in Gegenwart eines Salpetersäure/Schwefelsäure-Nitriersäuregemisches (MS) zu Dinitrotoluolen (R-DNT), insbesondere mit einem oder mehreren Reaktionsbehältern (Nitrierreaktoren) zur Durchführung der Nitrierung;
(ii) gegebenenfalls, in Produktionslinie stromabwärts zur Nitriereinrichtung (NE) angeordnet, mindestens eine Abtrenneinrichtung, insbesondere eine Scheideeinrichtung (Scheider), zur Abtrennung des Nitrierendsäuregemisches (SP) von den Rohdinitrotoluolen (R-DNT);
(iii) in Produktionslinie stromabwärts zur Nitriereinrichtung (NE) und zur gegebenenfalls vorhandenen Abtrenneinrichtung angeordnet, eine Vorrichtung (Anlage) zur Aufreinigung für die Rohdinitrotoluole (R-DNT), insbesondere eine Vorrichtung nach einem der vorangehenden Ansprüche, umfassend:
(a) mindestens eine Wascheinheit (WE) zur Wäsche der Rohdinitrotoluole (R-DNT) mit mindestens einem Waschmedium (WM); und,
(b) stromabwärts zur Wascheinheit (WE) angeordnet, mindestens eine Strippeinrichtung (Strippvorrichtung) (SK) zur Strippung (Strippgasbehandlung) der aus der Wascheinheit (WE) hervorgehenden gewaschenen Dinitrotoluole (W-DNT; DNT-I, DNT-II, DNT-III) mit mindestens einem gegenüber Dinitrotoluolen nichtreaktiven und/oder gegenüber Dinitrotoluolen inerten Gas (Strippgas) (SG), ausgewählt ist aus der Gruppe von Stickstoff, Sauerstoff, Edelgasen, Wasserstoff, Kohlenstoffoxiden und deren Mischungen, in Abwesenheit von Wasserdampf.

11. Vorrichtung nach einem der Ansprüche 7 bis 9 oder Produktionsanlage nach Anspruch 10,
wobei die Strippeinrichtung (SK) zusätzlich einen Wärmetauscher (WM1) zur Temperierung der gewaschenen Dinitrotoluole (W-DNT; DNT-I, DNT-II, DNT-III) vor Einbringung in die Strippeinrichtung (SK) umfasst; und/oder
wobei die Strippeinrichtung (SK) zusätzlich einen Wärmetauscher (WM2) zur Temperierung des Strippgases (SG) vor Einbringung in die Strippeinrichtung (SK) umfasst; und/oder
wobei, stromabwärts zur Strippeinrichtung (SK) angeordnet, eine Abgasbehandlungseinrichtung für die Aufreinigung und/oder Behandlung des mit aus den Dinitrotoluolen entfernten Verunreinigungen beladenen Strippgases vorgesehen ist, insbesondere wobei die Abgasbehandlungseinrichtung eine Wascheinrichtung (WK), insbesondere eine Waschkolonne, gegebenenfalls mit nachgeschalteter, vorzugsweise thermischer Abgasbehandlungsanlage (AB), umfasst oder insbesondere wobei die Abgasbehandlungseinrichtung eine vorzugsweise thermische Abgasbehandlungsanlage (AB) umfasst.

## Claims

1. A method for purifying crude dinitrotoluenes resulting from the nitration of toluene in the presence of a nitric acid/sulphuric acid nitrating acid compound, particularly to also remove particularly volatile impurities from the crude dinitrotoluenes,
**characterised in that**
(a) the crude dinitrotoluenes after separation of the nitrating acid compound initially undergo a washing with at least one washing agent, followed by a separation of the washing agent; and
(b) **in that** subsequently the washed dinitrotoluene resulting from process step (a) undergoes a stripping (stripping gas treatment) with at least one gas (stripping gas), wherein a gas being non-reactive to dinitrotoluenes and/or inert to dinitrotoluenes is used as the stripping gas, wherein the stripping gas is chosen from the group of nitrogen, oxygen, noble gases, hydrogen, carbon oxides and their compounds, and wherein the stripping is carried out in the absence of water vapour.

2. The method according to claim 1,
wherein in process step (a) the washing is carried out as liquid-liquid extraction; and/or
wherein in process step (a) the washing is carried out in one or a number of washing steps, particularly in one, two or three washing steps, particularly wherein each washing step can comprise one or a number of washing and/or extraction stages and/or particularly wherein each washing step can be carried out as a single stage or a number of stages, particularly as a single stage, as two stages or three stages; and/or
wherein in process step (a) the washing is carried out in three washing steps, wherein the washing in three washing steps comprises a first acid washing step (acid washing step"), a subsequent second basic (alkaline) washing step ("basic or alkaline wash") and a final third neutral washing step ("neutral washing step"); and/or
wherein in process step (a), the washing, particularly in each washing step, is carried out in the transverse current or in the counter current or in a combination of transverse and counter current, preferably by recycling the washing agent; and/or
wherein in process step (a), the separation of the washing agent occurs by means of a separating device (separator), particularly by means of a static separating device, or by means of a centrifugal separator.

3. The method according to one of the preceding claims,
wherein in process step (b) the stripping is carried out in a stripping device, particularly wherein a stripping column is used as the stripping device, particularly a single stage or multi-stage stripping column, preferably a tower-packing, sieve plate, bubble tray or falling film column or combination thereof, or a thin-film evaporator, a gas-liquid reactor or a horizontal stripping device; and/or
wherein in process step (b) the stripping is carried out in the transverse and/or counter current, preferably in the counter current, and/or wherein in process step (b) the stripping gas is conducted in the transverse and/or counter current, preferably in the counter current; and/or
wherein in process step (b) the stripping gas is carried out as a number of stages, particularly wherein in the different stripping stages, the stripping is carried out respectively in the transverse or counter current, preferably in the counter current and/or particularly wherein in the different stripping stages the stripping gas is conducted in the transverse or counter current, preferably in the counter current.

4. The method according to one of the preceding claims,
wherein in process step (b) the stripping is carried out at temperatures above the melting point of the dinitrotoluenes, particularly at a temperature of at least 56 °C, preferably at a temperature of at least 60 °C, more preferably at a temperature of at least 65 °C and/or wherein in process step (b) the stripping is carried out in a temperature range of 56 °C to 130 °C, particularly in a temperature range of 65 °C to 105 °C, preferably in a temperature range of 70 °C to 95 °C; and/or
wherein in process step (b) the stripping gas is used with a stripping gas temperature in the range of 65 °C to 140 °C, particularly in the range of 70 °C to 120 °C, preferably in the range of 70 °C to 95 °C and/or is exposed to the dinitrotoluenes and/or wherein in process step (b) the stripping gas upon feeding into the stripping appliance has a temperature in the range of 65 °C to 140 °C, particularly in the range of 70 °C to 120 °C, preferably in the range of 70 °C to 95 °C; and/or
wherein in process step (b), preferably during the entire method, the dinitrotoluenes are in the liquid aggregate state and/or above their melting point and/or wherein in process step (b), preferably during the entire method, the dinitrotoluenes have a temperature of at least 60 °C and more preferably a temperature in the range of 56 °C to 130 °C, particularly in the range of 60 °C to 105 °C, preferably in the range of 65 °C to 95 °C, particularly preferably in the range of 65 °C to 85 °C; and/or
wherein in process step (b) the stripping is carried out at the atmospheric pressure (normal pressure; 1.01325 bar), in a vaccuum (low pressure) or at high pressure, preferably at atmospheric pressure, and/or wherein in process step (b) the stripping is carried out at an absolute pressure in the range of 0.2 bar to 3 bar, particularly in the range of 0.3 bar to 1.2 bar, preferably in the range of 0.4 bar to 1.1 bar, particularly preferably at about 1 bar.

5. The method according to one of the preceding claims,
wherein in process step (b) the used volume of stripping gas is selected and/or adjusted depending on the conditions of the stripping, particularly temperature and/or pressure and/or duration of the stripping and type of stripping gas, and/or depending on the content and/or type of impurities in the dinitrotoluenes to be treated in process step (b) and/or depending on the desired residual content of impurities in the dinitrotoluenes resulting after process step (b); and/or
wherein in process step (b) the used volume of stripping gas, relating to 1 tonne of dinitrotoluenes to be treated and calculated as volumes of stripping gas in standard cubic meters under standard conditions (pressure of 1.01325 bar; air humidity of 0%, i.e. dry stripping gas; temperature of 0 °C [normal conditions according to DIN 1343, STPD]), is 0.1 to 1,000 standard cubic meters of stripping gas, particularly 0.5 to 500 standard cubic meters, preferably 1 to 200 standard cubic meters, particularly preferably 2 to 120 standard cubic meters, very particularly preferably 5 to 80 standard cubic meters, even more preferably 10 to 60 standard cubic meters; and/or
wherein in process step (b) the used volume of stripping gas, relating to 1 tonne of dinitrotoluenes to be treated and calculated as volumes of nitrogen as stripping gas under standard conditions (pressure of 1.01325 bar; air humidity of 0%, i.e. dry stripping gas; temperature of 0 °C [normal conditions according to DIN 1343, STPD]), is 0.5 to 500 kg, particularly 1 to 250 kg, preferably 1 to 140 kg, particularly preferably 5 to 50 kg, even more preferably 10 to 40 kg; and/or
wherein in process step (b) (likewise) a reduction of the water content of the treated dinitrotoluenes occurs, particularly wherein the water content in the dinitrotoluenes resulting after particularly preferably (b) is no more than 1 wt.-%, particularly no more than 0.8 wt.-%, preferably no more than 0.5 wt.-%, particularly preferably no more than 0.4 wt.-%, very particularly preferably no more than 0.3 wt.-%, even more preferably no more than 0.2 wt.-%, respectively in relation to the dinitrotoluenes; and/or
wherein in process step (b) the content of water in the resulting dinitrotoluenes is lowered such that no further water can be separated upon cooling the liquid dinitrotoluenes to their solidification point; and/or
wherein in process step (b) the stripping occurs following a preceding acidic, basic or neutral washing according to process step (a); and/or
wherein after executing process step (b) the stripping gas being charged with the impurities removed from the dinitrotoluenes undergoes a gas treatment, particularly a thermal gas treatment, and/or
wherein after executing process step (b) the stripping gas being charged with the impurities removed from the dinitrotoluenes undergoes a treatment to remove the impurities from the charged stripping gas, preferably by means of condensation and/or washing with a preferably aqueous washing agent, followed by a recycling of the purified stripping gas in process step (b) where necessary.

6. The method according to one of the preceding claims,
wherein the impurities to be removed from the crude dinitrotoluenes by means of the method are selected from the group of mineral acids, particularly sulphuric acid, nitric acid and nitrous acid, non-reacted and/or partially reacted reactants, nitrogen oxides, carbon oxides and nitrated and non-nitrated reaction by-products and degradation and oxidation products of the products and by-products originated from the nitration; and/or
wherein the impurities to be removed from the crude dinitrotoluenes by means of the method are selected from the group of (i) mineral acids, particularly sulphuric acid, nitric acid and nitrous acid; (ii) organic acids, particularly formic acid, acetic acid, oxalic acid, nitrobenzoic acid, such as mono-nitrobenzoic acid and di-nitrobenzoic acid, picric acid, hydrocyanic acid or the like; (iii) nitroaromatics, particularly nitrophenols, such as mono-nitrophenols, di-nitrophenols and tri-nitrophenols, and nitrocresols, such as mono-nitrocresols, di-nitrocresols and tri-nitrocresols; (iv) nitrogen oxides (nitric oxides), particularly nitrogen monooxide (NO), nitrogen dioxide (NO₂) and nitrous oxide (N₂O); (v) carbon oxides, particularly carbon monoxide (CO) and carbon dioxide (CO₂); (vi) hydrocarbons, particularly aliphatic and cycloaliphatic hydrocarbons; (vii) non-reacted or partially reacted reactants, particularly toluene; and/or
wherein the volatile impurities to be removed from the crude dinitrotoluenes have a higher volatility, particularly a higher vapour pressure and/or a lower flash point, than the crude dinitrotoluenes to be purified, particularly relating to the vapour pressure of the dinitrotoluenes under standard conditions (pressure of 1.01325 bar; air humidity of 0% [standard conditions according to DIN 1343, STPD]) and/or particularly relating to the flash point of the dinitrotoluenes under standard conditions (pressure of 1.01325 bar; air humidity of 0% [standard conditions according to DIN 1343, STPD]).

7. A device (system/plant) for purifying crude dinitrotoluenes resulting from the nitration of toluene in the presence of a nitric acid/sulphuric acid nitrosulfuric acid compound, after separation of the nitrating acid compound, particularly to also remove particularly volatile impurities from the crude dinitrotoluenes, particularly a device (system/plant) for executing a method according to one of the preceding claims,
wherein the device comprises:
(a) at least one washing unit (WE) for washing the crude dinitrotoluenes (R-DNT) with at least one washing agent (WM); and,
(b) arranged downstream to the washing unit (WE), at least one stripping device (stripping appliance) (SK) for stripping (stripping gas treatment) the washed dinitrotoluenes (W-DNT; DNT-I, DNT-II, DNT-III) resulting from the washing unit (WE) with at least one gas (stripping gas) (SG) being non-reactive to dinitrotoluenes and/or inert to dinitrotoluenes is selected from the group of nitrogen, oxygen, noble gases, hydrogen, carbon oxides and their compounds, in the absence of water vapour.

8. The device according to claim 7,
wherein the washing unit (WE) has one or a number, particularly one, two or three, preferably three, washing devices (WS-I, WS-II, WS-III) for washing the crude dinitrotoluenes (R-DNT), particularly one washing device (WS-I) for executing an acidic washing and/or one washing device (WS-II) for executing an alkaline washing and/or one washing device (WS-III) for executing a neutral washing; and/or
wherein the washing unit (WE) has three washing devices (WS-I, WS-II, WS-III) for washing the crude dinitrotoluenes (R-DNT), comprising one washing device (WS-I) for executing an acidic washing, a washing device (WS-II) for executing an alkaline washing being arranged downstream of the washing device (WS-I) for executing the acidic washing and one washing device (WS-III) for executing a neutral washing being arranged downstream of the washing device (WS-II) for executing the alkaline washing; and/or
wherein the washing unit (WE) additionally comprises at least one separating device for separating the used washing agent (WA) and/or the washing agent (WL) being charged with impurities removed from the dinitrotoluenes from the washed dinitrotoluenes (W-DNT; DNT-I, DNT-II, DNT-III).

9. The device according to claim 7 or 8,
wherein the stripping device (SK), particularly a stripping column, has a lower end (base) (KS), particularly a column sump, and an upper end (upper section) (KK), particularly a column head, and comprises a feeding device for the stripping gas (SG) being preferably arranged on the lower end (KS) and a feeding device for the washed dinitrotoluenes (W-DNT; DNT-I, DNT-II, DNT-III) preferably being arranged on the upper end (KK); and/or
wherein the stripping device (SK) is formed as a stripping column with a column sump (KS) as the lower end (base) and a column head (KK) as the upper end (upper section), particularly wherein the stripping column comprises a feeding device for the stripping gas (SG) being preferably arranged on the column sump (KS) and a feeding device for the washed dinitrotoluenes (W-DNT; DNT-I, DNT-II, DNT-III) being preferably arranged on the column head (KK); and/or
wherein the stripping device (SK) is designed such that the stripping device (SK) in the operating state facilitates a counter and/or transverse current flow, preferably a counter current flow, of washed dinitrotoluenes (W-DNT; DNT-I, DNT-II, DNT-III) on one side and stripping gas (SG) on the other.

10. A production plant for manufacturing dinitrotoluenes, particularly a production plan for the nitration (dinitration) of toluene to dinitrotoluenes in the presence of a nitric acid/sulphuric acid nitrating acid compound with subsequent purification of the crude dinitrotoluenes arising from the nitration,
wherein the production plant comprises:
(i) a nitrating device (NE) for the nitration (dinitration) of toluene (T) in the presence of a nitric acid/sulphuric acid nitrating acid compound (MS) to dinitrotoluenes (R-DNT), particularly with one or a number of reaction vessels (nitrating reactors) for executing the nitration;
(ii) where necessary, being arranged in the production line downstream of the nitrating device (NE), at least one separating device, particularly a separation device (separator), for the separation of the nitrating acid compound (SP) from the crude dinitrotoluenes (R-DNT);
(iii) being arranged in the production line downstream of the nitrating device (NE) and downstream of the possibly existing separating device, a device (system) for purifying crude dinitrotoluenes (R-DNT), particularly a device according to one of the preceding claims, comprising:
(a) at least one washing unit (WE) for washing the crude dinitrotoluenes (R-DNT) with at least one washing agent (WM); and,
(b) arranged downstream to the washing unit (WE), at least one stripping device (stripping appliance) (SK) for stripping (stripping gas treatment) the washed dinitrotoluenes (W-DNT; DNT-I, DNT-II, DNT-III) resulting from the washing unit (WE) with at least one gas (stripping gas) (SG) being non-reactive to dinitrotoluenes and/or inert to dinitrotoluenes is selected from the group of nitrogen, oxygen, noble gases, hydrogen, carbon oxides and their compounds, in the absence of water vapour.

11. The device according to one of claims 7 to 9 or the production plan according to claim 10,
wherein the stripping device (SK) also comprises a heat exchanger (WM1) for tempering the washed dinitrotoluenes (W-DNT; DNT-I, DNT-II, DNT-III) before transfer into the stripping device (SK); and/or
wherein the stripping device (SK) also comprises a heat exchanger (WM2) for tempering the stripping gas (SG) before transfer into the stripping device (SK); and/or
wherein, being arranged downstream of the stripping device (SK), a gas treatment device is provided for the purification and/or treatment of the stripping gas being charged with the impurities removed from the dinitrotoluenes, particularly wherein the gas treatment device comprises a washing device (WK), particularly a washing column, where necessary with a downstream, preferably thermal gas treatment system (AB), or particularly wherein the gas treatment device comrpises a preferably thermal gas treatment system (AB).

## Revendications

1. Procédé de purification de dinitrotoluènes bruts obtenus par la nitration du toluène en présence d'un mélange sulfonitrique acide nitrique/acide sulfurique, en particulier d'élimination simultanée, à partir des dinitrotoluènes bruts, en particulier des impuretés volatiles
**caractérisé en ce que**
(a) les dinitrotoluènes bruts sont, après séparation du mélange sulfonitrique, d'abord soumis à un lavage avec au moins un milieu de lavage, l'opération étant suivie d'une séparation du milieu de lavage ; et
(b) **en ce que** les dinitrotoluènes lavés, obtenus dans l'étape de procédé (a), sont soumis à une extraction (traitement par un gaz d'extraction) avec au moins un gaz (gaz d'extraction), le gaz d'extraction utilisé étant un gaz non réactif vis-à-vis des dinitrotoluènes et/ou inerte vis-à-vis des dinitrotoluènes, le gaz d'extraction étant choisi dans le groupe consistant en l'azote, l'oxygène, les gaz rares, l'hydrogène, les oxydes de carbone et les mélanges de ceux-ci, et l'extraction étant mise en oeuvre en l'absence de vapeur d'eau.

2. Procédé selon la revendication 1,
dans l'étape de procédé (a), le lavage étant mis en oeuvre sous forme d'une extraction liquide/liquide ; et/ou
dans l'étape de procédé (a), le lavage étant mis en oeuvre en une ou plusieurs étapes, en particulier en une, deux ou trois étapes, en particulier chaque étape de lavage pouvant comprendre un ou plusieurs étages de lavage et/ou d'extraction, et/ou en particulier chaque étape de lavage pouvant être mise en oeuvre dans un ou plusieurs étages, en particulier dans un étage, deux étages ou trois étages ; et/ou
dans l'étape de procédé (a), le lavage étant mis en oeuvre en trois étapes de lavage, le lavage en trois étapes de lavage comprenant une première étape de lavage acide ("étape de lavage acide"), une deuxième étape de lavage basique (alcaline) venant ensuite ("étape de lavage basique ou alcaline") et, pour terminer, une troisième étape de lavage neutre ("étape de lavage neutre") ; et/ou
dans l'étape de procédé (a), le lavage, en particulier dans chaque étape de lavage, étant mis en oeuvre à courant transversal ou à contre-courant, ou selon une combinaison d'un courant transversal et d'un contre-courant, de préférence avec recyclage du milieu de lavage ; et/ou
dans l'étape de procédé (a), la séparation du milieu de lavage s'effectuant à l'aide d'un dispositif séparateur (séparateur), en particulier d'un dispositif séparateur statique, ou à l'aide d'un séparateur centrifuge.

3. Procédé selon l'une des revendications précédentes,
dans l'étape de procédé (b), l'extraction est mise en oeuvre dans un dispositif d'extraction, par utilisation en particulier, en tant que dispositif d'extraction, d'une colonne d'extraction, en particulier d'une colonne d'extraction à un ou plusieurs étages, de préférence une colonne à garnissage, à plateaux perforés, à plateaux à cloches ou à film tombant, ou des combinaisons de celles-ci, ou encore en tant qu'évaporateur à couche mince, réacteur gaz/liquide, ou dispositif d'extraction horizontale ; et/ou
dans l'étape de procédé (b), l'extraction étant effectuée à courant transversal et/ou à contre-courant, de préférence à contre-courant, et/ou, dans l'étape de procédé (b), le gaz d'extraction étant guidé à courant transversal et/ou à contre-courant, de préférence à contre-courant ; et/ou
dans l'étape de procédé (b), l'extraction étant réalisée en plusieurs étages, en particulier, dans chacun des différents étages d'extraction, l'extraction étant réalisée à courant transversal ou à contre-courant, de préférence à contre-courant, et/ou en particulier, dans chacun des différents étages d'extraction, le gaz d'extraction étant guidé à courant transversal ou à contre-courant, de préférence à contre-courant.

4. Procédé selon l'une des revendications précédentes,
dans l'étape de procédé (b), l'extraction étant mise en oeuvre à des températures supérieures à la température de fusion des dinitrotoluènes, en particulier à une température d'au moins 56 °C, de préférence à une température d'au moins 60 °C, préférentiellement à une température d'au moins 65 °C, et/ou, dans l'étape de procédé (b), l'extraction étant mise en oeuvre sur une plage de températures de 56 °C à 130 °C, en particulier sur une plage de températures de 65 °C à 105 °C, de préférence sur une plage de températures de 70 °C à 95 °C ; et/ou
dans l'étape de procédé (b), le gaz d'extraction étant utilisé à une température du gaz d'extraction comprise dans la plage de 65 °C à 140 °C, en particulier dans la plage de 70 °C à 120 °C, de préférence dans la plage de 70 °C à 95 °C, et/ou étant mis en contact avec les dinitrotoluènes, et/ou, dans l'étape de procédé (b), le gaz d'extraction, lors de son introduction dans le dispositif d'extraction, présentant une température comprise dans la plage de 65 °C à 140 °C, en particulier dans la plage de 70 °C à 120 °C, de préférence dans la page de 70 °C à 95 °C ; et/ou
dans l'étape de procédé (b), de préférence pendant la totalité du procédé, les dinitrotoluènes se présentant à l'état d'agrégats liquides et/ou au-delà de leur température de fusion, et/ou, dans l'étape de procédé (b), de préférence pendant la totalité du procédé, le dinitrotoluène présentant une température d'au moins 60 °C et de préférence une température comprise dans la plage de 56 °C à 130 °C, en particulier dans la plage de 60 °C à 105 °C, de préférence dans la plage de 65 °C à 95 °C, d'une manière particulièrement préférée dans la plage de 65 °C à 85 °C ; et/ou
dans l'étape de procédé (b), l'extraction étant mise en oeuvre sous la pression atmosphérique (pression normale ; 1,01325 bar), sous vide (dépression) ou en surpression, de préférence sous la pression atmosphérique, et/ou, dans l'étape de procédé (b), l'extraction étant mise en oeuvre sous une pression absolue comprise dans la plage de 0,2 bar à 3 bar, en particulier dans la plage de 0,3 bar à 1,2 bar, de préférence dans la plage de 0,4 bar à 1,1 bar, d'une manière particulièrement préférée sous environ 1 bar.

5. Procédé selon l'une quelconque des revendications précédentes,
dans l'étape de procédé (b), la quantité utilisée de gaz d'extraction étant choisie et/ou ajustée en fonction des conditions de l'extraction, en particulier de la température et/ou de la pression et/ou de la durée de l'extraction, et/ou de la nature du gaz d'extraction, et/ou en fonction de la concentration et de la nature des impuretés dans les dinitrotoluènes à traiter dans l'étape de procédé (b), et/ou en fonction de la teneur résiduelle recherchée des impuretés dans les dinitrotoluènes résultant de l'étape de procédé (b) ; et/ou
dans l'étape de procédé (b), la quantité utilisée du gaz d'extraction, rapportée à 1 tonne des dinitrotoluènes à traiter, et calculée par le volume de gaz d'extraction par mètre cube normal dans des conditions normalisées (pression de 1,01325 bar ; humidité de l'air de 0 %, c'est-à-dire gaz d'extraction sec ; température de 0 °C [conditions normales selon DIN 1343, STPD]), étant de 0,1 à 1000 mètres cubes normaux de gaz d'extraction, en particulier de 0,5 à 500 mètres cubes normaux, de préférence de 1 à 200 mètres cubes normaux, d'une manière particulièrement préférée de 2 à 120 mètres cubes normaux, d'une manière tout particulièrement préférée de 5 à 80 mètres cubes normaux, d'une manière encore plus préférée de 10 à 60 mètres cubes normaux ; et/ou
dans l'étape de procédé (b), la quantité utilisée de gaz d'extraction, rapportée à 1 tonne de dinitrotoluènes à traiter, est calculée par la quantité d'azote, en tant que gaz d'extraction, dans des conditions normalisées (pression de 1,01325 bar ; humidité de l'air de 0 %, c'est-à-dire gaz d'extraction sec ; température de 0 °C [conditions normales selon DIN 1343, STPD]), étant de 0,5 à 500 kg, en particulier de 1 à 250 kg, de préférence de 1 à 140 kg, d'une manière particulièrement préférée de 5 à 50 kg, d'une manière encore plus préférée de 10 à 40 kg ; et/ou
dans l'étape de procédé (b) (d'une manière similaire), une réduction de la teneur en eau des dinitrotoluènes traités étant réalisée, en particulier la teneur en eau, dans les dinitrotoluènes résultant de l'étape de procédé (b), étant d'au plus 1 % en poids, en particulier d'au plus 0,8 % en poids, de préférence d'au plus 0,5 % en poids, d'une manière particulièrement préférée d'au plus 0,4 % en poids, d'une manière tout particulièrement préférée d'au plus 0,3 % en poids, d'une manière encore plus préférée d'au plus 0,2 % en poids, dans chaque cas par rapport aux dinitrotoluènes ; et/ou
dans l'étape de procédé (b), la teneur en eau des dinitrotoluènes obtenus étant abaissée de telle sorte que, lors du refroidissement des dinitrotoluènes liquides jusqu'à leur point de solidification, il n'y ait plus d'eau qui se sépare ; et/ou
dans l'étape de procédé (b), l'extraction étant réalisée après un lavage acide, basique ou neutre préalable selon l'étape de procédé (a) ; et/ou
après mise en oeuvre de l'étape de procédé (b), le gaz d'extraction chargé des impuretés éliminées des dinitrotoluènes étant soumis à un traitement des effluents gazeux, en particulier à un traitement thermique des effluents gazeux, et/ou
après mise en oeuvre de l'étape de procédé (b), le gaz d'extraction chargé des impuretés éliminées des dinitrotoluènes étant soumis à un traitement destiné à éliminer les impuretés du gaz d'extraction chargé, de préférence par condensation et/ou lavage avec un milieu de lavage de préférence aqueux, l'opération étant éventuellement suivie d'un recyclage du gaz d'extraction purifié dans l'étape de procédé (b).

6. Procédé selon l'une des revendications précédentes,
les impuretés éliminées des dinitrotoluènes bruts à l'aide du procédé étant choisies dans le groupe consistant en les acides minéraux, en particulier l'acide sulfurique, l'acide nitrique et l'acide nitreux, les réactifs n'ayant pas réagi et/ou ayant subi une réaction partielle, les oxydes d'azote, les oxydes de carbone et les sous-produits de réaction nitrés et non-nitrés, ainsi que les produits de dégradation et d'oxydation des produits et sous-produits provenant de la nitration ; et/ou
les impuretés à éliminer des dinitrotoluènes bruts à l'aide du procédé étant choisies dans le groupe consistant en (i) les acides minéraux, en particulier l'acide sulfurique, l'acide nitrique et l'acide nitreux, (ii) les acides organiques, en particulier l'acide formique, l'acide acétique, l'acide oxalique, les acides nitrobenzoïques tels que les acides mono- et dinitrobenzoïques, l'acide picrique, l'acide cyanhydrique ou analogues ; (iii) les composés nitroaromatiques, en particulier les nitrophénols tels que les mono-, di- et trinitrophénols, et les nitrocrésols tels que les mono-, les di- et les trinitrocrésols ; (iv) les oxydes d'azote (oxydes d'azote) en particulier le monoxyde d'azote (NO), les dioxyde d'azote (NO₂) et le protoxyde d'azote (N₂O) ; (v) les oxydes de carbone, en particulier le monoxyde de carbone (CO) et le dioxyde de carbone (CO₂) ; (vi) les hydrocarbures, en particulier les hydrocarbures aliphatiques et cycloaliphatiques ; (vii) les réactifs n'ayant pas réagi ou ayant subi une réaction partielle, en particulier le toluène ; et/ou
les impuretés volatiles à éliminer des dinitrotoluènes bruts présentant une humidité plus élevée, en particulier une pression de vapeur plus élevée et/ou un point d'ébullition plus bas, que les dinitrotoluènes bruts à purifier, en particulier par rapport à la pression de vapeur des dinitrotoluènes dans des conditions normalisées (pression de 1,01325 bar ; humidité de l'air de 0 % [conditions normales selon DIN 1343, STPD]) et/ou en particulier par rapport au point d'ébullition des dinitrotoluènes dans des conditions normalisées (pression de 1,01325 bar ; humidité de l'air de 0 % [conditions normales selon DIN 1343, STPD]).

7. Dispositif (installation) pour la purification de dinitrotoluènes bruts résultant de la nitration du toluène en présence d'un mélange sulfonitrique acide nitrique/acide sulfurique, après séparation du mélange sulfonitrique, en particulier pour l'élimination simultanée d'impuretés, en particulier volatiles, des dinitrotoluènes bruts, en particulier dispositif (installation) pour la mise en oeuvre d'un procédé selon l'une des revendications précédentes,
le dispositif comprenant :
(a) au moins une unité de lavage (WE), pour le lavage des dinitrotoluènes bruts (R-DNT) avec au moins un milieu de lavage (WM) ; et
(b) disposé en aval de l'unité de lavage (WE), au moins un équipement d'extraction (dispositif d'extraction) (SK) pour l'extraction (traitement par un gaz d'extraction) des dinitrotoluènes lavés (W-DNT ; DNT-I, DNT-II, DNT-III) provenant de l'unité de lavage (WE), avec au moins un gaz non réactif vis-à-vis des dinitrotoluènes et/ou inerte vis-à-vis des dinitrotoluènes (gaz d'extraction) (SG), choisi dans le groupe consistant en l'azote, l'oxygène, les gaz rares, l'hydrogène, les oxydes de carbone et les mélanges de ceux-ci, en l'absence de vapeur d'eau.

8. Dispositif selon la revendication 7,
l'unité de lavage (WE) présentant un ou plusieurs, en particulier un, deux ou trois, de préférence trois équipements de lavage (WS-I, WS-II, WS-III) pour le lavage des dinitrotoluènes bruts (R-DNT), en particulier un équipement de lavage (WS-I) pour la mise en oeuvre d'un lavage acide et/ou un équipement de lavage (WS-II) pour la mise en oeuvre d'un lavage alcalin et/ou un équipement de lavage (WS-III) pour la mise en oeuvre d'un lavage neutre ; et/ou
l'unité de lavage (WE) présentant trois équipements de lavage (WS-I, WS-II, WS-III) pour le lavage des dinitrotoluènes bruts (R-DNT), comprenant un équipement de lavage (WS-I) pour la mise en oeuvre d'un lavage acide, un équipement de lavage (WS-II), disposé en aval de l'équipement de lavage (WS-I) pour la mise en oeuvre du lavage acide, pour la mise en oeuvre d'un lavage alcalin, et un équipement de lavage (WS-III), disposé en amont de l'équipement de lavage (WS-II) pour la mise en oeuvre du lavage alcalin, pour la mise en oeuvre d'un lavage neutre ; et/ou
l'unité de lavage (WE) comprenant en outre au moins un équipement de séparation pour la séparation, d'avec les dinitrotoluènes lavés (W-DNT ; DNT-I, DNT-II, DNT-III), du milieu de lavage (WL) usé et/ou chargé des impuretés éliminées des dinitrotoluènes.

9. Dispositif selon la revendication 7 ou 8,
l'équipement d'extraction (SK), en particulier une colonne d'extraction, présentant une extrémité inférieure (partie inférieure) (KS), en particulier un fond de colonne, et une extrémité supérieure (partie supérieure) (KK), en particulier une tête de colonne, et comprenant un équipement, disposé de préférence au niveau de l'extrémité inférieure (KS) destiné à amener le gaz d'extraction (SG) et un équipement disposé au niveau de l'extrémité supérieure (KK), destiné à amener les dinitrotoluènes lavés (W-DNT ; DNT-I, DNT-II, DNT-III) ; et/ou
l'équipement d'extraction (SK) étant conçu comme une colonne d'extraction, avec un fond de colonne (KS) en tant qu'extrémité inférieure (partie inférieure) et une tête de colonne (KK) en tant qu'extrémité supérieure (partie supérieure), en particulier la colonne d'extraction comprenant un dispositif disposé de préférence au niveau du fond de colonne (KS), destiné à amener le gaz d'extraction (SG), et un équipement disposé de préférence au niveau de la tête de colonne (KK), destiné à amener les dinitrotoluènes lavés (W-DNT ; DNT-I, DNT-II, DNT-III) ; et/ou
l'équipement d'extraction (SK) étant conçu de telle sorte que l'équipement d'extraction (SK) permette, en état de fonctionnement, un écoulement à contre-courant et/ou à courant transversal, de préférence un écoulement à contre-courant, d'une part des dinitrotoluènes lavés (W-DNT; DNT-I, DNT-II, DNT-III), et d'autre part du gaz d'extraction (SG).

10. Installation de production pour la fabrication de dinitrotoluènes, en particulier installation de production pour la nitration (dinitration) de toluène en dinitrotoluènes en présence d'un mélange sulfonitrique acide nitrique/acide sulfurique, avec purification ultérieure des dinitrotoluènes bruts provenant de la nitration,
l'installation de production comprenant :
(i) un équipement de nitration (NE) pour la nitration (dinitration) de toluène (T) en dinitrotoluènes (R-DNT) en présence d'un mélange sulfonitrique (MS) acide nitrique/acide sulfurique, en particulier comportant un ou plusieurs réacteurs (réacteurs de nitration) pour la mise en oeuvre de la nitration ;
(ii) éventuellement, disposé dans la ligne de production en aval de l'équipement de nitration (NE), au moins un équipement de séparation, en particulier un équipement de séparation (séparateur), pour la séparation du mélange sulfonitrique (SP) des dinitrotoluènes bruts (R-DNT) ;
(iii) disposé dans la ligne de production en aval de l'équipement de nitration (NE) et de l'équipement de séparation éventuellement présent, un dispositif (installation) pour la purification des dinitrotoluènes bruts (R-DNT), en particulier un dispositif selon l'une des revendications précédentes, comprenant :
(a) au moins une unité de lavage (WE) pour le lavage des dinitrotoluènes bruts (R-DNT) à l'aide d'au moins un milieu de lavage (WM) ; et,
(b) disposé en aval de l'unité de lavage (WE), au moins un équipement d'extraction (dispositif d'extraction) (SK) pour l'extraction (traitement par un gaz d'extraction) des dinitrotoluènes lavés (W-DNT ; DNT-I, DNT-II, DNT-III) provenant de l'unité de lavage (WE), avec au moins un gaz (gaz d'extraction) (SG), non réactif vis-à-vis des dinitrotoluènes et/ou inerte vis-à-vis des dinitrotoluènes, choisi dans le groupe consistant en l'azote, l'oxygène, les gaz rares, l'hydrogène, les oxydes de carbone et les mélanges de ceux-ci, en l'absence de vapeur d'eau.

11. Dispositif selon l'une des revendications 7 à 9 ou installation de production selon la revendication 10,
l'équipement d'extraction (SK) comprenant en outre un échangeur de chaleur (WM1) pour porter à l'équilibre de température les dinitrotoluènes lavés (W-DNT ; DNT-I, DNT-II, DNT-III) avant introduction dans l'équipement d'extraction (SK) ; et/ou,
l'équipement d'extraction (SK) comprenant en outre un échangeur de chaleur (WM2) pour porter à l'équilibre de température le gaz d'extraction (SG) avant introduction dans l'équipement d'extraction (SK) ; et/ou
disposé en aval de l'équipement d'extraction (SK), un équipement de traitement des effluents gazeux étant prévu pour la purification et/ou le traitement du gaz d'extraction chargé en impuretés éliminées des dinitrotoluènes, en particulier l'équipement de traitement des effluents gazeux comprenant un équipement de lavage (WK), en particulier une colonne de lavage, éventuellement avec une installation (AB) montée en aval, de préférence thermique, de traitement des effluents gazeux, ou en particulier l'équipement de traitement des effluents gazeux comprenant une installation (AB) de préférence thermique de traitement des effluents gazeux.
